# EUROPEAN PATENT APPLICATION

(11) **EP 4 484 451 A1**
(43) Date of publication of application: **01.01.2025**
(21) Application number: 23759617.6
(22) Date of filing: 02.02.2023
(51) Int. Cl.: C08B 15/06, A61Q 1/00, A61Q 3/00, A61Q 15/00, A61Q 17/04, A61Q 19/00, C08K 5/05, C08L 1/08, C08L 83/04, C08L 91/00, A61Q 5/00, A61K 8/04, A61K 8/73

(54) **HYDROPHOBIC CELLULOSE NANOFIBERS, HYDROPHOBIC CELLULOSE NANOFIBER LIQUID DISPERSION, AND COSMETIC**

(30) Priority: 22.02.2022 JP 2022025196
(71) Applicant: SHIN-ETSU CHEMICAL CO., LTD., Tokyo 1000005 (JP)
(72) Inventor: ABE, Takuya, Annaka-shi, Gunma 379-0224 (JP)
(74) Representative: Sonnenhauser, Thomas Martin
(86) International application number: PCT/JP2023/003317
(87) International publication number: WO 2023/162611

(57) **Abstract**

The present invention relates a hydrophobic cellulose nanofiber including (A) a cellulose nanofiber and (B) isocyanate group-containing organopolysiloxane, wherein the isocyanate group-containing organopolysiloxane is bonded to a hydroxy group of the cellulose nanofiber through a urethane bond. By this, it is possible to provide hydrophobic cellulose nanofibers that can be dispersed in liquid oils at room temperature, especially in silicone oils, and a dispersion medium for the same.

## Description

### TECHNICAL FIELD

The present invention relates to a hydrophobic cellulose nanofiber, a hydrophobic cellulose nanofiber dispersion, and a cosmetic.

### BACKGROUND ART

Water-soluble polymers are used in the formulation of cosmetics to change feeling on use of the product by thickening a water and to increase the stability of the formulation by preventing powder from settling. Water-soluble polymers are broadly classified into natural polymers such as xanthan gum and tamarind gum, semi-synthetic polymers such as hydroxyethyl cellulose, and synthetic polymers such as polyacrylic acid. The use of water-soluble polymers poses problems in that they become sticky after application to impair feeling on use and that a sufficient thickening effect cannot be obtained depending on presence of salt or pH of the formulation. In recent years, cellulose nanofibers have attracted attention as a water-soluble polymer. It is characterized by non-sticky feeling on use and fresh feeling on use thanks to thixotropic properties, and the above problems can be solved (Patent Document 1). In addition, because it exhibits excellent properties such as high strength, high elastic modulus, and low coefficient of thermal expansion, it has been used in applications other than cosmetics such as a gas barrier material and a reinforcing material for polymeric materials (Patent Document 2).

In order to maximize the above properties, it is necessary to unravel cellulose fibers finely down to nano-size. As a method for it, it is possible to prepare a uniform and transparent cellulose nanofiber aqueous dispersion due to electric charge repulsion between nanofibers and water osmotic pressure effects, which is obtained by introducing an anionic functional group onto the surface of cellulose fiber. Examples of such anionic functional groups include carboxyl groups, carboxymethyl groups, sulfate ester groups, phosphate ester groups, phosphite ester groups, xanthate ester groups, etc., and various studies have been conducted to date (Patent Documents 3, 4, 5).

On the other hand, because cellulose nanofibers have many hydrophilic groups on their surfaces, most of dispersion media are limited to water. Therefore, when it is heated and melted with a resin that has low polarity and poor compatibility, it aggregates in the resin, resulting in lower strength than the original resin. In addition, they have poor thickening properties in dispersion media other than water, making it difficult to improve the feel or the emulsion stability of formulations, which are represented by water-in-oil type emulsions. Therefore, the challenge is to disperse cellulose nanofibers in various organic solvents other than water. However, in organic solvents, the degree of ionization of the anionic functional groups present on the surface of cellulose nanofibers is low, and therefore, electric charge repulsion is weakened, resulting in that hydrogen bonds between hydroxy groups are prioritized to make aggregation easily.

Patent Document 6 discloses that neutralizing carboxyl group-containing cellulose nanofibers with long-chain monoalkylamine makes it possible to disperse them in a mixed solvent of acetone:water = 1:1 or isopropanol. However, dispersion in less polar solvents, especially in silicone oil, has not been achieved. Further, Patent Document 7 discloses that neutralizing carboxyl group-containing cellulose nanofibers with polyetheramine makes it possible to thicken toluene, methylene chloride, and ethylene glycol used as a dispersion medium and to be dispersed therein. However, dispersion in silicone oil has not been achieved. Further, Patent Document 8 discloses that using an aqueous dispersion of carboxyl group-containing cellulose nanofibers neutralized with a monoamine improved the emulsion stability when an oily material is blended into a formulation. However, the dispersion medium is water, and dispersion into other dispersion media has not been achieved.

Cellulose nanofibers treated with silane or silicone are also known. In Patent Document 9 discloses that emulsion stability of oil-in-water type emulsion was improved by mixing a carboxyl group-containing cellulose nanofiber aqueous dispersion, low-viscosity dimethyl silicone oil, and amino-modified silicone, and by using cellulose nanofibers hydrophobized by silicone in the formulation. However, there is no description that hydrophobic cellulose nanofibers are used in water-in-oil type emulsion. Patent Document 10 discloses that uniform dispersion into a resin or rubber is possible by hydrophobizing a part of hydroxy groups on the surface of carboxyl group-containing cellulose nanofibers with alkoxysilane or silazane. However, dispersion in organic solvents or silicone oil has not been achieved. Patent Document 11 discloses that dispersion in toluene or tetrahydrofuran is possible by treating carboxyl group-containing cellulose nanofibers replaced with a polar organic solvent with amino-modified silicone, but dispersion in silicone oil has not been achieved.

As mentioned above, there are examples of dispersing cellulose nanofibers in dispersion media other than water using various approaches, but dispersibility in low polarity oils such as silicone oil still remains an issue. Furthermore, when replacing water as a dispersion medium with an organic solvent in order to easily perform hydrophobization treatment, electric charge repulsion becomes weaker, making aggregation more likely to occur. Cellulose nanofibers that once aggregated are difficult to defibrate, and challenges still remain in approaching methods for hydrophobization without forming aggregates. Furthermore, although there were cases using cellulose nanofibers in oil-in-water type emulsions, there is no cases applying cellulose nanofibers to water-in-oil type emulsions and it was unclear whether they contributed to emulsion stability.

### CITATION LIST

### PATENT LITERATURE

Patent Document 1: JP 2014-141675 A
Patent Document 2: JP 2009-057552 A
Patent Document 3: JP 2017-071700 A
Patent Document 4: JP 2013-127141 A
Patent Document 5: JP 2018-141249 A
Patent Document 6: JP 2012-021081 A
Patent Document 7: JP 2017-019896 A
Patent Document 8: JP 2012-126786 A
Patent Document 9: JP 2021-095557 A
Patent Document 10: JP 2020-128476 A
Patent Document 11: JP 2020-164670 A

### SUMMARY OF INVENTION

### TECHNICAL PROBLEM

The present invention has been made in view of the above circumstances. An object of the present invention is to provide hydrophobic cellulose nanofibers that can be dispersed in a liquid oil agent at room temperature, particularly in silicone oil, and a dispersion thereof. Furthermore, it is an object of the present invention to provide a cosmetic that is excellent in applicability and fresh feeling on use, and has good stability of the formulation over time.

### SOLUTION TO PROBLEM

To solve the above problems, the present invention provides a hydrophobic cellulose nanofiber comprising:
(A) a cellulose nanofiber; and
(B) isocyanate group-containing organopolysiloxane,
wherein the isocyanate group-containing organopolysiloxane is bonded to a hydroxy group of the cellulose nanofiber through a urethane bond.

Such hydrophobic cellulose nanofibers can be dispersed in liquid oils at room temperature, especially in silicone oils.

Moreover, in the present invention, the component (A) cellulose nanofiber preferably further comprises an anionic functional group.

Such hydrophobic cellulose nanofibers are preferred because they exhibit high dispersibility.

In this event, 0.2 mass% aqueous dispersion of the cellulose nanofiber having the anionic functional group has preferably a 600 nm light transmittance of 80% or more and the cellulose nanofiber having the anionic functional group in the aqueous dispersion has preferably a fiber width of 300 nm or less.

Such hydrophobic cellulose nanofibers are preferable because the light transmittance of the dispersion does not decrease and the transparency of the film is maintained.

Moreover, in the present invention, the anionic functional group is preferably selected from the group consisting of a carboxyl group, a carboxymethyl group, a sulfate ester group, a phosphate ester group, a phosphite ester group, and a xanthate ester group.

Such hydrophobic cellulose nanofibers are preferable because the cellulose nanofibers are easily defibrated, a dispersion with high light transmittance can be easily obtained, and solubility in water can be restrained.

Moreover, in the present invention, the component (B) isocyanate group-containing organopolysiloxane is preferably represented by the following general formula (1) or (2), 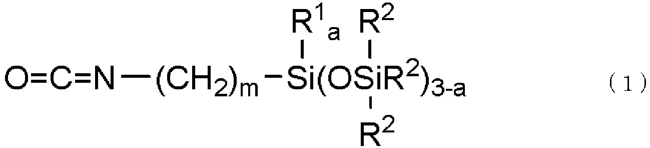 wherein R¹ is a monovalent alkyl group having 1 to 6 carbon atoms; R² is independently an alkyl group having 1 to 6 carbon atoms, a phenyl group, or a triorganosiloxy group represented by -OSiR³₃; R³ is independently a monovalent organic group having 1 to 6 carbon atoms; "m" is an integer of 1 to 10; and "a" is an integer of 0 to 1, 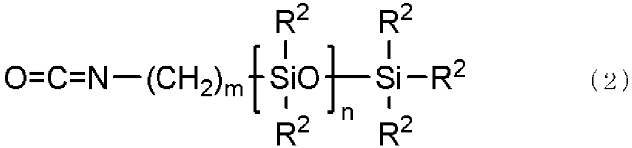 wherein R² is the same as the above; "m" is an integer of 1 to 10; and "n" is an integer of 1 to 500.

Such hydrophobic cellulose nanofibers can further improve the effects of the present invention.

In this event, the isocyanate group-containing organopolysiloxane represented by the general formula (1) is preferably tris(trimethylsiloxy)silylpropyl isocyanate.

Such hydrophobic cellulose nanofibers can further improve the effects of the present invention.

Moreover, the present invention provides a hydrophobic cellulose nanofiber dispersion, in which the hydrophobic cellulose nanofiber described in the above is dispersed in a component (C) oil agent.

Such a hydrophobic cellulose nanofiber dispersion can be dispersible in a liquid oil agent at room temperature, particularly in silicone oil.

In this event, the component (C) oil agent is preferably one or more kinds selected from the group consisting of aliphatic alcohols, silicone oils, and hydrocarbon oils.

In the present invention, such an oil agent can be used for a hydrophobic cellulose nanofiber dispersion.

Moreover, in the present invention, a mass ratio between the component (C) oil agent and the hydrophobic cellulose nanofiber preferably falls in 1:0.0001 to 1:0.5.

Such a hydrophobic cellulose nanofiber dispersion is preferable from the viewpoint of usability because the concentration of cellulose nanofibers does not become too high and the viscosity does not become high.

Moreover, in the present invention, 0.2 mass% oily dispersion of the hydrophobic cellulose nanofiber preferably has a 600 nm light transmittance of 70% or more.

Such a hydrophobic cellulose nanofiber dispersion is preferable because it has sufficient transparency, does not contain many aggregates, and does not have low reactivity.

Moreover, the present invention provides a cosmetic comprising the hydrophobic cellulose nanofiber dispersion described in the above.

Such cosmetics can be excellent in applicability and fresh feeling on use and can have a good stability of the formulation over time.

In this event, the hydrophobic cellulose nanofiber dispersion is preferably used as an emulsified composition.

Such cosmetics can further improve the effects of the present invention.

### ADVANTAGEOUS EFFECTS OF INVENTION

The inventive hydrophobic cellulose nanofibers can be easily dispersed in a specific dispersion medium, and when the dispersion medium evaporates, a highly transparent and flexible film can be formed. A cosmetic containing this hydrophobic cellulose nanofiber dispersion is a cosmetic that gives good feeling on use, has good cosmetic persistence, good spreadability and good finish.

In particular, by using cellulose nanofibers having an anionic functional group as a material, it is possible to obtain a dispersion liquid with few aggregates to give a high thickening property for oil agents. Furthermore, by changing the bonding ratio of isocyanate group-containing organopolysiloxane to the hydroxy groups of cellulose nanofibers, it is possible to control the thickening properties of the oil agent. Therefore, by blending the inventive hydrophobic cellulose nanofiber dispersion into cosmetics as an agent for improving feel, it is possible to provide cosmetics with very good feel and over time stability. Furthermore, because there are few aggregates, it is possible to form a highly transparent film by evaporating a dispersion medium. When the obtained film is incorporated into a cosmetic, the resultant cosmetic does not become sticky when applied, gives good feeling on use, is excellent in water resistance and durability, and has adhesivity to the skin. In addition, this cosmetic has durability of makeup, spreads well, and has good finish. Furthermore, it exhibits excellent functions as a viscosity imparting agent and a dispersion stabilizer in various applications such as pharmaceuticals, agricultural chemicals, toiletry products, spray products, and paints.

### DESCRIPTION OF EMBODIMENTS

As mentioned above, there has been a need for the development of hydrophobic cellulose nanofibers that can be dispersed in liquid oils at room temperature, especially in silicone oils, and a dispersion medium for the same.

The present inventor has found out that cellulose nanofibers can be dispersed in a dispersion medium other than water by hydrophobization with isocyanate group-containing organopolysiloxane and that this hydrophobic cellulose nanofiber forms a film when the dispersion medium is volatilized. Furthermore, the present inventor has found out also that the dispersion medium is thickened by the hydrophobic cellulose nanofibers and can be used as an agent for improving feel and as a stabilizer.

Further, when modifying cellulose nanofibers with an isocyanate group-containing compound, the absence of water in the reaction system is preferable because it causes side reactions. However, because cellulose nanofibers are highly hydrophilic, they are usually in a form of an aqueous dispersion. Therefore, when the water in a dispersion medium is replaced with an organic solvent or the like, electric charge repulsion becomes weaker to make aggregation easier. However, by replacing it with a liquid water-soluble organic compound having a boiling point of 100°C or higher at 25°C at 1 atm, it is possible to obtain a cellulose nanofiber dispersion without forming aggregates. With this method, water can be removed while preventing aggregation, and a highly transparent organic solvent dispersion of cellulose nanofibers can be obtained. Therefore, the reaction between the hydroxy group of cellulose nanofibers and the isocyanate group can be performed without a side reaction. The inventor found out that the obtained hydrophobic cellulose nanofiber dispersion can solve the above problems and completed the present invention.

That is, the present invention is a hydrophobic cellulose nanofiber comprising:
(A) a cellulose nanofiber; and
(B) isocyanate group-containing organopolysiloxane,
wherein the isocyanate group-containing organopolysiloxane is bonded to a hydroxy group of the cellulose nanofiber through a urethane bond.

The present invention is described below in detail, but the present invention is not limited to the examples described below.

The present invention is a hydrophobic cellulose nanofiber, wherein the component (B) isocyanate group-containing organopolysiloxane is bonded to a hydroxy group of the component (A) cellulose nanofiber through a urethane bond. Each will be explained in detail below.

### (A) Cellulose nanofiber

The cellulose nanofiber is a fine cellulose fiber obtained by defibrating plant materials such as pulp fibers. It is composed of cellulose microfibrils alone or an aggregate thereof, and has a fiber width of 3 to 100 nm, an aspect ratio of 10 or more, and a length of 100 um or less. Examples of cellulose raw materials for producing cellulose nanofibers include chemical pulps represented by hardwood kraft pulp and softwood kraft pulp, mechanical pulps represented by stone ground pulp and thermoground pulp, and waste paper pulps represented by waste of unbleached kraft paper, waste newspapers, waste leaflets, etc. Depending on the purpose of the present invention, one of these materials may be used alone or two or more kind thereof may be used in combination.

For defibrating cellulose fibers, known methods can be used. There are two main types of treatment: mechanical treatment and chemical treatment, and the method is not limited.

Defibration methods using mechanical treatment include, for example, the grinder method in which grinding is performed between grinding stones, the microfluidizer method in which pressurized materials are collided with each other at high speed to be defibrated by collision force, pressure difference, and microcavitation, the pulverization method with a ball mill, a bead mill, or the like, and the twin-screw kneading method in which materials are kneaded into a resin.

In recent years, defibration technology using chemical treatment has been developed for the purpose of reducing the energy required for manufacturing. As for the defibration method using chemical treatment, for example, an anionic functional group can be introduced by subjecting cellulose fibers as a material to a chemical reaction. The introduction of the functional group causes electric charge repulsion among the nanofibers and the osmotic pressure effect of water, making it possible to prepare a uniform and transparent aqueous dispersion of the cellulose nanofibers with low energy.

The cellulose nanofibers used as a material in the present invention preferably have a smaller fiber diameter (fiber width) because a highly dispersed cellulose nanofiber has better reactivity with isocyanate group-containing organopolysiloxane. In particular, defibration by a chemical treatment is preferable because it can disperse highly with low energy.

It is preferable that the component (A) cellulose nanofiber used in the present invention further has an anionic functional group. The anionic functional group can be introduced by subjecting cellulose fiber as a material to a chemical reaction.

It is preferable that cellulose nanofibers having an anionic functional group are capable of high dispersion due to repulsion between anionic groups and that its 0.2 mass% aqueous dispersion has a 600 nm light transmittance of 80% or more. 85% or more is more preferable, and 90% or more is further preferable. When it is 80% or more, the dispersion has sufficient transparency and few aggregates, and its reactivity does not decrease. The upper limit of the light transmittance at 600 nm is not particularly limited, but it may be, for example, 98% or less. Moreover, it is preferable that the average fiber diameter (fiber width) is 300 nm or less. 100 nm or less is more preferable, and 80 nm or less is further preferable. When it is 300 nm or less, the light transmittance of the dispersion does not decrease and the transparency of the film will be maintained. The lower limit of the average fiber diameter (fiber width) is not particularly limited, but it may be, for example, 2 nm or more. Further, the average fiber length is preferably 0.1 um or more and 1,000 um or less, more preferably 0.1 um or more and 800 um or less, and further preferably 0.1 um or more and 600 um or less. Within this range, the thickening effect is not impaired and the strength increase easily when compounded with a resin.

In the present invention, the light transmittance is the transmittance obtained by measuring a direct transmitted light at a wavelength of 600 nm using an ultraviolet-visible spectrophotometer.

A cellulose nanofiber dispersion liquid with a solid content concentration of 0.01 mass% is prepared, dropped onto a copper grid, and dried. The dried dispersion is used as a sample to be observed using a transmission electron microscope. By observing three or more images in which the fibers do not overlap, fiber diameters and fiber lengths of 20 fibers per image are calculated. The average values thereof are defined as the average fiber diameter and the average fiber length.

The average aspect ratio (axial ratio, fiber length/fiber width) of cellulose nanofibers is preferably 50 or more. Its upper limit is not particularly limited, but it is preferably 20,000 or less. When the aspect ratio is 50 or more, it is fibrous, and the dispersion has sufficient thickening properties and is stable. When the aspect ratio is 20,000 or less, the viscosity of the dispersion does not become too high and it is easy to be handled. The average aspect ratio can be calculated according to the formula: average aspect ratio = average fiber length/average fiber diameter.

The hydrophobic cellulose nanofibers preferably have a crystal structure of cellulose type I because natural cellulose is used as a material. The degree of crystallinity is 65% or more, preferably 70% or more, from the viewpoint of exhibit of film properties or strength. The crystal structure of the type I can be identified from the peaks in the spectral window ranged from around 2θ = 14 to 17° and 22 to 23° in the profile obtained by wide-angle X-ray diffraction.

The crystallinity of cellulose nanofibers is a value measured by an X-ray diffraction method in accordance with "General rules for X-ray diffraction analysis" of JIS K0131:1996. Cellulose nanofibers are in amorphous state and in crystalline state, and the ratio of ones in the crystalline state is expressed as crystallinity.

Examples of the anionic functional group to be introduced include those selected from the group consisting of a carboxy group, a carboxymethyl group, a sulfate ester group, a phosphate ester group, a phosphite ester group, and a xanthate ester group. These functional groups can be introduced into cellulose nanofibers by known methods. Alternatively, commercially available modified cellulose nanofibers into which an anionic functional group has already been introduced may be used.

The amount of the anionic functional group to be introduced is optimized depending on the type of its functional group. Specifically, the following ranges are preferable. Within the following range, cellulose nanofibers are easily defibrated, a dispersion liquid with high light transmittance is easily obtained, and solubility in water can also be restrained.

For example, in the case of a carboxy group, it is preferably 0.6 to 2.0 mmol/g, more preferably 1.0 to 2.0 mmol/g, based on the weight of the carboxylated cellulose nanofiber. In addition, in the case of carboxymethyl groups, it is preferably 0.6 to 2.0 mmol/g, more preferably 1.0 to 2.0 mmol/g, based on the weight of the carboxylated cellulose nanofibers. Further, in the case of sulfate ester groups, it is preferably 0.4 to 3.0 mmol/g, more preferably 0.5 to 3.0 mmol/g, and particularly preferably 0.5 to 2.0 mmol/g, based on the weight of sulfate-esterified cellulose nanofibers. Furthermore, in the case of phosphate ester groups, it is preferably 0.1 to 2.0 mmol/g, more preferably 0.2 to 1.5 mmol/g, based on the weight of cellulose nanofibers containing a phosphate ester group. Moreover, in the case of a phosphite ester group, it is preferably 0.1 to 3.5 mmol/g, more preferably 0.6 to 1.8 mmol/g, and particularly preferably 0.9 to 1.5 mmol/g, based on the weight of the phosphite-esterified cellulose nanofiber. And then, in the case of xanthate ester groups, it is preferably 0.3 to 1.2, more preferably 0.3 to 1.0, per glucose unit.

The amount of the anionic functional group introduced into cellulose nanofibers can be measured, for example, by conductometric titration. This is the method to measure the amount introduced by dropping an alkaline solution such as a sodium hydroxide aqueous solution, a potassium hydroxide aqueous solution, or the like into slurry or dispersion containing cellulose nanofibers. For example, when the anionic functional group is a carboxyl group and the alkaline solution is an aqueous sodium hydroxide solution, the amount of carboxyl groups can be calculated from the amount of sodium hydroxide required for neutralization.

Counter ions of the introduced anionic functional group include alkali metal ions such as lithium, sodium, and potassium; alkaline earth metal ions such as calcium and magnesium; hydrogen ions; aliphatic ammonium; and aromatic ammonium. One of these may be applied alone, or two or more kinds thereof may be applied in combination. Cations of sodium and potassium are preferable.

Cellulose nanofibers containing an anionic functional group preferably use a water-soluble organic compound or water as a dispersion medium. Examples of water-soluble organic compounds include methanol, ethanol, etc.

The anionic functional group is preferably a carboxyl group, a phosphate ester group, or a phosphite ester group from the viewpoint of efficiency of introducing a modification group into cellulose nanofibers and efficient dispersion into silicone, and a phosphate ester group or a phosphite ester group are more preferable. Phosphate groups and phosphorous groups bond with a hydroxyl group and an oxo group, have a high negative electric charge, have a strong repulsion between cellulose nanofibers, resulting in easy defibration. Further, from the viewpoint of capable of suppressing yellowing of cellulose nanofibers, a phosphite ester group is more preferable. Phosphate groups have more hydrogen ions to be dissociated than phosphorous acid groups, so the pH is lower. It is thought that the lower the pH, the more likely double bonds will be formed in cellulose due to a Maillard reaction or the reduction reaction, and the easier it will yellow.

In the present invention, the bonding manner of organopolysiloxane to cellulose nanofibers is urethane bonding. The urethane bond is formed by a reaction between a hydroxy group of the cellulose nanofiber and an isocyanate group of the organopolysiloxane in the presence of a catalyst.

### (B) Isocyanate group-containing organopolysiloxane

The component (B) isocyanate group-containing organopolysiloxane is preferably represented by the following general formula (1) or (2).

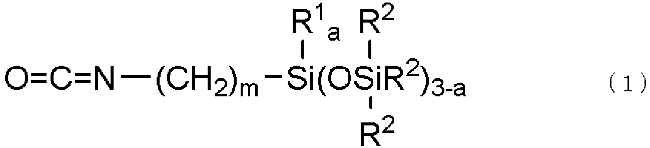

In the formula, R¹ is a monovalent alkyl group having 1 to 6 carbon atoms; R² is independently an alkyl group having 1 to 6 carbon atoms, a phenyl group, or a triorganosiloxy group represented by -OSiR³₃; R³ is independently a monovalent organic group having 1 to 6 carbon atoms; "m" is an integer of 1 to 10; and "a" is an integer of 0 to 1.

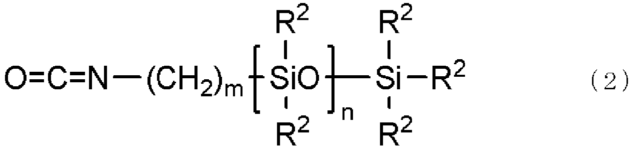

In the formula, R² is the same as the above; "m" is an integer of 1 to 10; and "n" is an integer of 1 to 500.

In the above formula (1), R¹ is a monovalent alkyl group having 1 to 6 carbon atoms, and the specific examples thereof include: alkyl groups such as a methyl group, an ethyl group, a propyl group, and a butyl group; and cycloalkyl groups such as a cyclopentyl group and a cyclohexyl group. Further, R² is independently an alkyl group having 1 to 6 carbon atoms, a phenyl group, or a group represented by -OSiR³₃, in which R³ is independently a monovalent organic group having 1 to 6 carbon atoms. The alkyl group having 1 to 6 carbon atoms are exemplified as the same as described for the above R¹. The examples of groups represented by -OSiR³₃ include a trimethylsiloxy group, an ethyldimethylsiloxy group, a phenyldimethylsiloxy group, a vinyldimethylsiloxy group, a chloromethyldimethylsiloxy group, a 3,3,3-trifluoropropyldimethylsiloxy group, etc.

In the above general formula (1), "a" is an integer of 0 to 1, and "m" is an integer of 1 to 10. In a preferred embodiment of the above general formula (1), "m" is 3, R¹ and R² are all methyl groups, and "a" is 0, is preferable.

"n" in the above general formula (2) is an integer of 1 to 500, preferably 1 to 100, and more preferably 1 to 50. Within this range, the weight ratio occupied by organopolysiloxane is not high, and the properties derived from cellulose nanofibers does not deteriorate.

In the present invention, the component (B) isocyanate group-containing organopolysiloxane may be a commercially available product or may be synthesized according to a known method. One kind may be used alone, or two or more kinds may be used in combination.

The component (B) isocyanate group-containing organopolysiloxane preferably has a kinematic viscosity of 1 mm²/s or more and 500 mm²/s or less, and more preferably 1 mm²/s or more and 100 mm²/s or less, when measured at 25°C with a Cannon-Fenske viscometer described in JIS Z8803:2011. When within this range, it is preferable in terms of usability and reactivity.

In particular, as the component (B) isocyanate group-containing organopolysiloxane, it is preferable to use tristrimethylsiloxysilylpropyl isocyanate represented by the following formula (3), that is the case where "m" = 3; R² is a methyl group; and "a" is 0 in the above general formula (1). When the tristrimethylsiloxysilylpropyl isocyanate and the cellulose nanofibers are reacted, hydroxy groups in the cellulose nanofibers react and hydrophobic cellulose nanofibers represented by the following general formula (4) can be obtained.

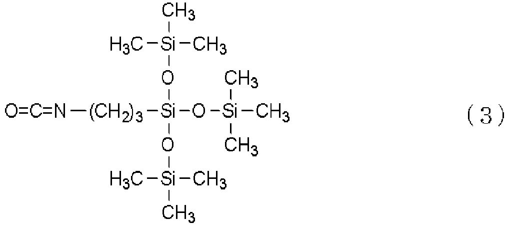

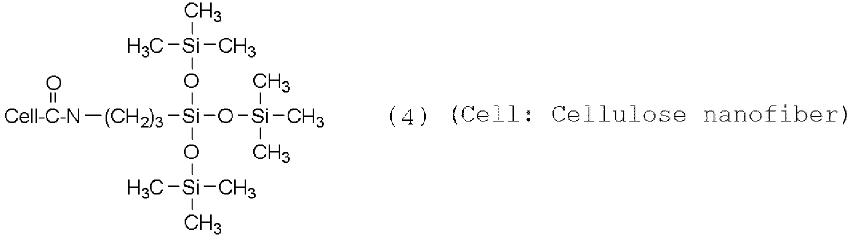

For example, when the cellulose nanofiber is a phosphite ester-containing cellulose nanofiber, a hydroxy group at any one of the positions C2, C3, and C6 is modified via a urethane bond.

### (C) Oil agent

The hydrophobic cellulose nanofibers are preferably the hydrophobic cellulose nanofiber that is dispersed in a component (C) oil agent. The component (C) oil agent preferably contains one or more kinds selected from the group consisting of aliphatic alcohols, silicone oils, and hydrocarbon oils.

The aliphatic alcohols include: monohydric aliphatic alcohols represented by methanol, ethanol, 1-propanol, 2-propanol, 1-butanol, 2-methyl-1-propanol, 2-butanol, 2-methyl-2-propanol, 1-pentanol, 2-methylbutanol, 2-pentanol, 1-hexanol, 2-methylpentanol, 1-heptanol, 1-octanol, 1-nonanol, 1-decanol, etc.; and dihydric aliphatic alcohols represented by ethylene glycol, 1,2-propylene glycol, etc.

The hydrocarbon oil includes: aromatic hydrocarbons represented by toluene, xylene, etc.; aliphatic ketones such as acetone, methyl ethyl ketone, diethyl ketone, methyl isobutyl ketone, etc.; aliphatic hydrocarbons represented by hexane, heptane, octane, cyclohexane, isooctane, isododecane, isohexadecane, etc.

The silicone oils described above include linear or branched organopolysiloxanes having low to high viscosity represented by dimethylpolysiloxane (manufactured by Shin-Etsu Chemical Co., Ltd.: KF-96L-1cs, KF-96L-1.5cs, KF-96L-2cs, etc.), octamethyltetrasiloxane (D4), decamethylpentasiloxane (D5) (manufactured by Shin-Etsu Chemical Co., Ltd.: KF-995), dodecamethylhexasiloxane (D6), tristrimethylsiloxymethylsilane (manufactured by Shin-Etsu Chemical Co., Ltd.: TMF-1.5), caprylyl methicone, phenyl trimethicone, methylphenylpolysiloxane (manufactured by Shin-Etsu Chemical Co., Ltd.: KF-54, KF-54HV), diphenylsiloxyphenyl trimethicone (manufactured by Shin-Etsu Chemical Co., Ltd.: KF-56A), methylhexylpolysiloxane, dimethylsiloxane-methylphenylsiloxane copolymers, etc.

Among the above oil agents, it is preferable to use silicone oil.

The mass ratio of the component (C) oil agent to the hydrophobic cellulose nanofibers is preferably 1:0.0001 to 1:0.5. When it is within this range, the concentration of cellulose nanofibers does not become too high and the viscosity does not become high, which is preferable from the viewpoint of usability.

0.2 mass% aqueous dispersion of the hydrophobic cellulose nanofiber preferably has a 600 nm light transmittance of 70% or more, more preferably 80% or more, and further preferably 90% or more. When it is 70% or more, transparency is sufficient, there are few aggregates, and reactivity does not decrease. The upper limit of the light transmittance at 600 nm is not particularly limited, but may be, for example, 98% or less.

Moreover, the average fiber diameter is preferably 300 nm or less, more preferably 100 nm or less, and further preferably 80 nm or less. When it is 300 nm or less, the light transmittance of the dispersion liquid does not decrease and transparency of the film is maintained. Further, the average fiber length is preferably 0.1 um or more and 1,000 um or less, more preferably 0.1 um or more and 800 um or less, and further preferably 0.1 um or more and 600 um or less. Within this range, the thickening effect is not impaired and it is easy to increase the strength when incorporated into a resin.

### [Production Method]

Because cellulose nanofibers are hydrophilic, water-soluble polar solvents represented by water or alcohol are used as a dispersion medium. When manufacturing the hydrophobic cellulose nanofibers of the present invention, it is necessary to replace the dispersion medium because water and alcohol are reactive with isocyanate groups and are not suitable as reaction solvents. Therefore, it is preferable to first replace the dispersion medium with a hydrophilic dispersion medium containing less water or alcohol, then perform hydrophobization treatment, and then replace with the desired oil agent.

Regarding the embodiments described above, a method for manufacturing a hydrophobic cellulose nanofiber dispersion including the following Steps 1 to 3 will be described.
Step 1: In the aqueous dispersion of the component (A) cellulose nanofibers, water as a dispersion medium is replaced with the component (D) an organic solvent to obtain a cellulose nanofiber dispersion.
Step 2: The component (B) isocyanate group-containing organopolysiloxane is added to the dispersion obtained in Step 1 to hydrophobize the component (A) cellulose nanofibers in the dispersion.
Step 3: The organic solvent of the hydrophobic cellulose nanofiber dispersion obtained in Step 2 is replaced with the component (C) oil agent.

In Step 1, the component (D) organic solvent is the organic compound that is a water-soluble liquid having a boiling point of 100°C or higher and does not react with isocyanate group. The boiling point is the boiling point under 1 atmosphere, and is preferably 100°C or higher, more preferably 110°C or higher, and further preferably 150°C or higher. A boiling point of 100°C or higher is preferable because there is no fear that component (D) volatilize when removing moisture to cause aggregation of cellulose nanofibers.

The solubility of the component (D) in water is preferably 30 g or more, more preferably 50 g or more, per 100 g of water at 25°C. When it is 30 g or more, it is preferable because there is no fear that cellulose nanofibers will precipitate in the dispersion.

Specific examples of the component (D) include: amides such as N,N-dimethylacetamide, N,N-dimethylformamide, and N-methyl-2-pyrrolidone; heterocyclic compounds such as pyridine; and sulfoxides such as dimethylsulfoxide. One kind of them may be used alone or two or more kinds thereof may be used in combination. Among these, N-methyl-2-pyrrolidone is particularly preferable.

As the defibration device used in the dispersion of Step 1, any of the following devices can be used. For example, it is possible to use a household mixer, a homomixer in high-speed rotation, a disper, a low-pressure homogenizer, a high-pressure homogenizer, a ball mill, a jet mill, a twin-screw extruder, an ultrasonic dispersion apparatus, a grinder, or the like. Instead of the above apparatus, it is possible to use a defibrating apparatus commonly used for household use or industrial production. Among the above-mentioned defibrating apparatus, it is preferable to use a low-pressure homogenizer or a high-pressure homogenizer.

In performing the reaction between the hydroxy groups of the cellulose nanofibers and the isocyanate group-containing organopolysiloxane in Step 2, it is undesirable for a solvent reactive with the isocyanate groups to remain. Therefore, it is undesirable for water or alcohol to remain in the reaction system, and it is necessary to remove them sufficiently. In particular, because water as a dispersion medium has the effect of stably dispersing cellulose nanofibers, it is not preferable to remove water in the absence of another dispersion medium. Therefore, as a method for removing water selectively and replacing it with the component (D) organic solvent, it is preferable to distill water off under reduced pressure. The pressure is 100 mmHg or less, more preferably 50 mmHg or less.

The amount of water and alcohol contained in the cellulose nanofiber dispersion in which replacement with the component (D) was performed is preferably 1 mass% or less, more preferably 0.1 mass% or less. When it is 1 mass% or less, the isocyanate groups of the component (B) isocyanate group-containing organopolysiloxane are not deactivated, which is preferable because it does not cause generation of by-products.

The used amount of the component (D) organic solvent is preferably 10 to 90 mass%, more preferably 20 to 90 mass%, of the entirety of the reaction solution (system). When it is within the above range, uniformity of the reaction system is maintained and the reaction proceeds efficiently. When the concentration is 10 mass% or more, the cellulose nanofibers does not increase the viscosity of the component (D), and thus the viscosity does not become high and the reactivity does not decrease. When it is 90 mass% or less, the concentration is not decreased and the working efficiency is not lowered.

In Step 2, the mixing amount of the component (B) isocyanate group-containing organopolysiloxane is 2 to 60 mass% (weight of isocyanate group-containing organopolysiloxane/weight of cellulose nanofiber) with respect to the cellulose nanofibers contained in the component (A) cellulose nanofibers replaced with the component (D). It is preferable to be mixed in the range, more preferably 5 to 40 mass%. When it is 2 mass% or more, the isocyanate group-containing organopolysiloxane that has reacted with the hydroxy groups of the cellulose nanofibers is sufficient, so that dispersibility in the component (C) oil agent does not decrease. When it is 60 mass% or less, the hydroxy groups necessary for thickening the component (C) oil agent are not be blocked too much, so that the thickening property does not decrease.

Further, in Step 2, it is preferable to add a component (E) a urethanization catalyst.

Specific examples of the component (E) include amines such as triethylamine, triethylenediamine, and N-methylmorpholine, and known catalysts used for forming a urethane bond such as organic metal compounds such as stannous oleate and di-n-butyltin dilaurate. The added amount may be determined appropriately arbitrarily depending on the type and amount of the component (B) and reaction temperature.

In the urethane bond-forming reaction, conditions are not particularly limited, but the reaction temperature is preferably 25 to 100°C, more preferably 30 to 90°C, under reflux. When the reaction temperature is 100°C or lower, yellowing of cellulose or a decrease in the degree of polymerization will not occur. When it is 25°C or higher, there is no risk that the reaction rate decrease. The reaction time is preferably 1 to 10 hours with heating. The reaction time is preferably within this range because there is no possibility that the cellulose nanofibers yellow.

After the urethane bond-forming reaction, it is desirable to add water or a lower alcohol such as methanol or ethanol in order to deactivate the unreacted component (B) isocyanate group-containing organopolysiloxane. In particular, from the viewpoint of easy removal, it is preferable to use methanol, ethanol, or 2-propanol. The amount added is preferably 1 mass% or more and 10 mass% or less of the component (B), and more preferably 1 mass% or more and 8 mass% or less.

In the present invention, in Step 3, before replacing the component (D) with the component (C), it is preferable to add a step of washing the cellulose nanofiber dispersion having the component (D) as a dispersion medium with a component (F) organic compound that is liquid at 25°C and 1 atm.

By repeating washing and filtration of a cellulose nanofiber dispersion that contains the component (D) as a dispersion medium, using the component (F) organic compound that is liquid at 25°C and 1 atm, it is possible to obtain swollen gel that is the hydrophobic cellulose nanofibers swollen by the component (F).

Specific examples of the component (F) include: alcohols having 1 to 4 carbon atoms such as methanol, ethanol, n-propyl alcohol, 2-propanol, n-butyl alcohol, and isobutyl alcohol; ketones such as acetone, diethyl ketone, and methyl ethyl ketone; esters such as methyl acetate, ethyl acetate, and butyl acetate; aromatic hydrocarbons such as toluene and xylene; and ethers such as diethyl ether and tetrahydrofuran. In particular, from the viewpoint of easy removal, it is preferable to use methanol, ethanol, and 2-propanol.

By washing with the component (F), it is possible to remove the unreacted (B) isocyanate group-containing organopolysiloxane, by-products derived from the component (B), the component (D) organic solvent, and the component (E) urethanization catalyst.

In particular, the unreacted (B) isocyanate group-containing organopolysiloxane used as a material and the by-products derived from the component (B) are unstable as materials, and thus it is not undesirable that they remain. By washing with the component (F), the total amount of the remained is preferably reduced to 1 mass% or less, more preferably to 0.1 mass%, and further preferably to 0.01 mass% or less, with respect to the hydrophobic cellulose nanofibers.

In the present invention, by adding the component (C) oil agent and removing the component (F), it is possible to obtain a hydrophobic cellulose nanofiber dispersion replaced with the component (C).

As a method for selectively removing the component (F), distillation under reduced pressure is preferable. The pressure is preferably 100 mmHg or less, more preferably 50 mmHg or less. Additionally, when the component (C) oil agent having a lower boiling point than the component (F) is used, it is undesirable because the distillation under reduced pressure removes the component (C) also, which may cause the hydrophobic cellulose nanofibers to aggregate. It is also undesirable to add the component (C) to a dried or gelled product of the hydrophobic cellulose nanofiber in which entire or part of liquid components were removed from the dispersion of the component (F) because it is difficult to defibrate the aggregates and to disperse hydrophobic cellulose nanofibers uniformly in the component (C).

### [Physical Properties of Hydrophobic Cellulose Nanofiber]

The inventive hydrophobic cellulose nanofiber is solid and has film-forming properties. Because the transparency and continuity of the formed film decrease depending on the amount of aggregates contained in the dispersion, it is preferable that the light transmittance of the dispersion is 70% or more, more preferably 80% or more, and further preferably 90% or more. By being within the above range, it can be suitably used as a film forming agent.

Whether the inventive hydrophobic cellulose nanofibers exhibit film properties can be judged by whether a film is formed after first dropping 1.5g of the hydrophobic cellulose nanofiber dispersion diluted with the component (C) an oil agent on a polytetrafluoroethylene (PTFE) resin plate and then drying dispersion at 105°C for 3 hours. When a film is not formed, the appearance is not transparent and has powder floated to the surface. Therefore, when blended into cosmetics, it has poor followability with the skin, resulting in unnatural finish.

By incorporating it into cosmetics as a film-forming agent, it is possible to obtain cosmetics that is not sticky upon application and have good feeling on use. In addition, the formed film has excellent water resistance and durability, and has good adhesivity to the skin, so makeup lasts long and has excellent spreadability and finish.

Furthermore, the inventive hydrophobic cellulose nanofibers have the effect of thickening the dispersion medium by entangling the nanofibers with each other in the dispersion medium. Particularly when the dispersion medium is silicone, the thickening effect is high. Therefore, when a hydrophobic cellulose nanofiber dispersion is used as an emulsion composition, by thickening the oil phase, a cosmetic with very good feel and over time stability can be provided. Further, especially when used in a water-in-oil type emulsion composition, it can thicken the external phase, contributing to improved feel and stabilization.

### [Cosmetics]

Hydrophobic cellulose nanofibers and hydrophobic cellulose nanofiber dispersions of the present invention can be used for various purposes, and are particularly applicable for materials of all cosmetics used externally for skin and hair. Moreover, in that case, it is desirable to apply it in the form of a dispersion. That is, it can be a cosmetic containing the hydrophobic cellulose nanofiber dispersion described above. The amount of hydrophobic cellulose nanofibers to be blended is preferably in the range of 0.1 to 40 mass%, more preferably in the range of 0.1 to 20 mass% of the entirety of the cosmetic. When it is 0.1 mass% or more, a sufficient feel can be obtained, and when it is not more than 40 mass%, the feeling on use does not deteriorate.

The inventive hydrophobic cellulose nanofibers, the inventive hydrophobic cellulose nanofiber dispersion, and cosmetics containing the same may contain various other components used in ordinary cosmetics, as other components. The other components may include, for example, a component (G) that is oil agent other than the component (C), a component (H) powder, a component (I) surfactant, a component (J) crosslinked organopolysiloxane, a component (K) coating agent, a component (L) other additives. One of these may be used alone or two or more kinds thereof may be used in combination appropriately. These components are appropriately selected and used depending on the type, etc. of a cosmetic, and the amount thereof may be a known amount according to the type, etc. of a cosmetic.

### Component (G): Oil Agent Other than the Component (C)

Depending on the purpose, the inventive cosmetic may contain one or two or more oil agents selected from oil agents other than the component (C) as the component (G). Any oil agent in solid, semi-solid, or liquid can be used as long as they are commonly used in cosmetics. For example, it is possible to use natural animal and vegetable oils, semi-synthetic oils, hydrocarbon oils, silicone oils, higher alcohols, ester oils, fluorine-based oil agents, ultraviolet absorber, etc. When blending an oil other than the component (C), the amount of the oil agent other than the component (C) is not particularly limited, but is preferably 1 to 95 mass% of the entirety of the cosmetic, more preferably 1 to 30 mass%.

### ·Natural animal and vegetable oil agent and semi-synthetic oil agent

Natural animal and vegetable oil agents and semi-synthetic oil agents include: germ oils such as Persea Gratissima (Avocado) Oil (INCI), Linum Usitatissimum (Linseed) Seed Oil (INCI), Prunus Amygdalus Dulcis (Sweet Almond) Oil (INCI), perilla oil, Olea Europaea (Olive) Fruit Oil (INCI), Torreya Californica (California Nutmeg) Oil (INCI), Cymbopogon Nardus (Citronella) Oil (INCI), Kyounin Yu (INCI), Triticum Vulgare (Wheat) Germ Oil (INCI), Sesamum Indicum (Sesame) Seed Oil (INCI), Oryza Sativa (Rice) Germ Oil (INCI), Oryza Sativa (Rice) Bran Oil (INCI), Camellia Kissi Seed Oil (INCI), Carthamus Tinctorius (Safflower) Seed Oil (INCI), china wood oil, cinnamon oil, squalane, squalene, Glycine Soja (Soybean) Oil, Camellia Sinensis Seed Oil, Camellia Japonica Seed Oil (INCI), Oenothera Biennis (Evening Primrose) Oil (INCI), Zea Mays (Corn) Germ Oil (INCI), RAPE SHUSHI YU(JPN) (INCI), Japanese wood oil, Zea Mays (Corn) Germ Oil (INCI), persic oil (Labeling Name), Elaeis Guineensis (Palm) Oil (INCI), Elaeis Guineensis (Palm) Kernel Oil (INCI), Ricinus Communis (Castor) Seed Oil (INCI), Helianthus Annuus (Sunflower) Seed Oil (INCI), Vitis Vinifera (Grape) Seed Oil (INCI), Simmondsia Chinensis (Jojoba) Seed Oil (INCI), Macadamia Ternifolia Seed Oil (INCI), Limnanthes Alba (Meadowfoam) Seed Oil (INCI), Gossypium Herbaceum (Cotton) Seed Oil (INCI), Japan wax kernel oil, Cocos Nucifera (Coconut) Oil (INCI), and Arachis Hypogaea (Peanut) Oil (INCI); Natural animal oil such as Shark Liver Oil (INCI), Cod Liver Oil (INCI), Fish Liver Oil (INCI), Turtle Oil (INCI), Mink Oil (INCI), and Egg Oil (INCI); semi-synthetic oil and fat such as Hydrogenated Coconut Oil (INCI), hydrogenated castor oil (Labeling Name), Methyl Ricinoleate, and Lanolin Oil (INCI).

### · Hydrocarbon oil

Examples of the hydrocarbon oil include linear or branched hydrocarbon oils. The hydrocarbon oil may be a volatile hydrocarbon oil or a nonvolatile hydrocarbon oil. Specific examples thereof include olefin oligomer, Dodecane (INCI), Undecane (INCI), Squalane (INCI), Squalene (INCI), Mineral Oil (INCI), polyisobutylene (Labeling Name), Hydrogenated Polyisobutene (INCI), and C13-15 Alkane (INCI).

### · Silicone oil

Examples of the silicone oil include: silicone rubbers such as amino-modified organopolysiloxane, pyrrolidone-modified organopolysiloxane, pyrrolidonecarboxylic acid-modified organopolysiloxane, highly polymerized gummy Dimethicone (INCI), gummy amino-modified organopolysiloxane, a gummy dimethylsiloxane-methylphenylsiloxane copolymer; cyclic organopolysiloxane solutions of silicone gums or rubbers; amino acid-modified silicones, fluorine-modified silicones, silicone resins; and dissolved products of silicone resins.

### · Higher alcohols

The higher alcohols are, for example, preferably alcohols having 6 or more carbon atoms. Specific examples thereof include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, phytosterol, POE cholesterol ether, monostearyl glycerol ether (batyl alcohol), and monooleyl glyceryl ether (serakyl alcohol).

### · Ester oils

The ester oil is a liquid oil in the form of a condensation of a fatty acid having 1 to 20 carbon atoms and an alcohol having 1 to 20 carbon atoms, and includes polyesters such as monoesters, diesters, and triesters. Specific Examples include: Diisobutyl Adipate (INCI), Diheptylundecyl Adipate (INCI), n-alkyl glycol monoisostearates such as Isostearyl Isostearate (INCI); Isocetyl Isostearate (INCI), Trimethylolpropane Triisostearate (INCI), Glycol Diethylhexanoate (INCI), Cetyl Ethylhexanoate (INCI), Triethylhexanoin (INCI), Trimethylolpropane Triethylhexanoate (INCI), Pentaerythrityl Tetraethylhexanoate (INCI), Cetyl Ethylhexanoate (INCI), octyldodecyl esters such as Octyldodecyl Stearoyl Stearate (INCI); Oleyl Oleate (INCI), Octyldodecyl Oleate (INCI), Decyl Oleate (INCI), Neopentyl Glycol Diethylhexanoate (INCI), Neopentyl Glycol Dicaprate (INCI), Triethyl Citrate (INCI), Diethylhexyl Succinate (INCI), Amyl Acetate (INCI), Etyl Acetate (INCI), Butyl Aceetate (INCI), Isocetyl Stearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), palmitic acid esters such as Isopropyl Palmitate (INCI), Ethylhexyl Isopalmitate (INCI), and Isocetyl Palmitate, Hexyldecyl Palmitate (INCI); Cholesteryl Hydroxystearate (INCI), myristic acid esters such as Isopropyl Myristate (INCI), Octyldodecyl Myristate (INCI), and Myristyl Myristate (INCI); Ethylhexyl Laurate (INCI), Hexyl Laurate (INCI), Dioctyldodecyl Lauroyl Glutamate (INCI), Isopropyl Lauroyl Sarcosinate (INCI), Diisostearyl Malate (INCI), glyceride oils such as Glyceryl Acetate (INCI), and Glyceryl Stearate (INCI).

### ·Fluorine-based oil agent

Examples of the fluorine-based oil agent include: perfluoropolyethers such as Polyperfluoromethylisopropyl Ether (INCI); perfluorocarbons such as Perfluorodecalin (INCI) and Perfluorohexane (INCI); etc.

### ·Ultraviolet absorber

Examples of an ultraviolet absorber include Homosalate (INCI), Octocrylene (INCI), Butyl Methoxydibenzoylmethane (INCI), Ethylhexyl Salicylate (INCI), Diethylamino Hydroxybenzoyl Hexyl Benzoate (INCI), Benzophenone-6 (INCI), Benzophenone-9 (INCI), Benzophenone-1 (INCI), Polysilicone-15 (INCI), Ethylhexyl Dimethoxybenzylidene Dioxoimidazolidine Propionate (INCI), Benzophenone-2 (INCI), Terephthalylidene Dicamphor Sulfonic Acid (INCI), Ethylhexyl Triazone (INCI), Isopentyl Trimethoxycinnamate Trisiloxane (INCI), Drometrizole Trisiloxane (INCI), Ethylhexyl Dimethyl PABA (INCI), Isopropyl Methoxycinnamate (INCI), Ethylhexyl Methoxycinnamate (INCI), Bis-Ethylhexyloxyphenol Methoxyphenyl Triazine (INCI), Benzophenone-3 (INCI), Benzophenone-4 (INCI), Benzophenone-5 (INCI), Phenylbenzimidazole Sulfonic Acid (INCI), Methylene Bis-Benzotriazolyl Tetramethylbutylphenol (INCI), Glyceryl Ethylhexanoate Dimethoxycinnamate (INCI), Glyceryl PABA (INCI), Diisopropyl Methyl Cinnamate (INCI), Cinoxate (INCI), Ethylhexyl Dimethoxybenzylidene Dioxoimidazolidine Propionate (INCI), etc. Further, it is possible to use an UVA absorber such as Diethylamino Hydroxybenzoyl Hexyl Benzoate (INCI), and an UVB absorber such as Ethylhexyl Methoxycinnamate (INCI), together in any combination thereof.

### Component (H): Powder

The powder is not particularly limited as long as it is a material that can be normally blended into cosmetics, but examples thereof include pigments, silicone spherical powder, etc. When blending powder, the blended amount of powder is not particularly limited, but it is preferably blended in an amount of 0.1 to 90 mass%, more preferably 1 to 35 mass% of the entirety of the cosmetic.

The pigment is not particularly limited as long as it is commonly used in makeup cosmetics. Examples thereof include: inorganic pigments such as talc, mica, sericite, synthetic phlogopite, barium sulfate, aluminum oxide, kaolin, silica, calcium carbonate, zinc oxide, titanium oxide, red iron oxide, yellow iron oxide, black iron oxide, ultramarine, navy blue, carbon black, lower titanium oxide, cobalt violet, chromium oxide, chromium hydroxide, cobalt titanate, bismuth oxychloride, and titanium-mica pearl pigment; Organic pigments such as zirconium, barium, or aluminum lakes such as Red No. 201, Red No. 202, Red No. 204, Red No. 205, Red No. 220, Red No. 226, Red No. 228, Red No. 405, Orange No. 203, Yellow No. 205, Yellow No. 4, Yellow No. 5, Blue No. 1, Blue No. 404, and Green No. 3; natural pigments such as chlorophyll and β-carotene; and dyes.

Note that, as for the powder mentioned above, it is possible to use the powder of which the particle surface is treated. The surface treatment agents are preferably those can impart hydrophobicity from the viewpoint of water resistance of the formulation and are not particularly limited as long as they can impart hydrophobicity. Examples of treatment agents include: silicone treatments, waxes, paraffins, organic fluorine compounds such as phosphates with perfluoroalkyls; surfactants, amino acids such as N-acylglutamic acid; and metal soaps such as aluminum stearate and magnesium myristate. Silicone treatment agents are more preferable, and examples of them include: silanes or silylation agents such as caprylic silane (manufactured by Shin-Etsu Chemical Co., Ltd.: AES-3083) or trimethoxysilyl dimethicone; silicone oils such as dimethyl silicone (manufactured by Shin-Etsu Chemical Co., Ltd.: KF-96A series), methyl hydrogen type polysiloxane (manufactured by Shin-Etsu Chemical Co., Ltd.: KF-99P, KF-9901, etc.), silicone branched type silicone treatment agent (manufactured by Shin-Etsu Chemical Co., Ltd.: KF-9908, KF-9909, etc.); acrylic silicones (manufactured by Shin-Etsu Chemical Co., Ltd.: KP-574, KP-541), etc. Further, one of the surface hydrophobization treatment agents mentioned above may be used alone or two or more kinds thereof may be used in combination. Specific examples of surface-treated color pigments include the KTP-09 series manufactured by Shin-Etsu Chemical Co., Ltd., particularly KTP-09W, 09R, 09Y, and 09B. Specific examples of dispersions containing hydrophobized fine-particle titanium oxide or hydrophobized fine-particle zinc oxide include Shin-Etsu Chemical Co., Ltd.'s SPD-T5, T6, T7, T5L, Z5, Z6, Z5L, etc. When this component is blended, its content is preferably 0.01 to 95 mass% of the cosmetic.

Examples of silicone spherical powder include crosslinked silicone powder (i.e., so-called silicone rubber powder made of organopolysiloxane having a crosslinked structure of repeating chains of diorganosiloxane units), silicone resin particles (polyorganosilsesquioxane resin particles having a three-dimensional network structure), silicone resin-coated silicone rubber powder, etc. Specific examples of crosslinked silicone powder and silicone resin particles are known by names such as (dimethicone/vinyl dimethicone) crosspolymer, polymethylsilsesquioxane, etc. These are commercially available as powders or as swollen products containing silicone oil. For example, they are sold with product names such as KMP-598, KMP-590, KMP-591, KSG-016F, which are all manufactured by Shin-Etsu Chemical Co., Ltd. These powders impart slipperiness to cosmetics and improve the feeling on use due to the rolling effect unique to spherical powders. One of these may be used alone or two or more kinds may be used in combination.

Silicone resin-coated silicone rubber powder is particularly preferable in terms of the effect of improving feel such as preventing stickiness, the effect of correcting irregularities such as wrinkles and pores, etc. Specific examples of silicone rubber powder coated with silicone resin are defined by Japanese Labeling Name of Cosmetic Ingredient and known by names such as (vinyl dimethicone/methicone silsesquioxane) crosspolymer, (diphenyl dimethicone/vinyl diphenyl dimethicone/silsesquioxane) crosspolymer, polysilicone-22 and polysilicone-1 crosspolymer. These are commercially available under the trade names such as KSP-100, KSP-101, KSP-102, KSP-105, KSP-300, KSP-411, KSP-441, etc. These are all manufactured by Shin-Etsu Chemical Co., Ltd. One of these powders may be used alone or two or more kinds thereof may be used in combination. When this component is blended, it is preferable 0.01 to 95 mass% of a cosmetic.

### Component (I): Surfactant

The surfactant includes nonionic, anionic, cationic, and amphoteric surfactants. It is possible to use surfactants used for a general cosmetic, according to purposes of the present invention. Among these surfactants, preferable surfactants are partially crosslinked polyether-modified silicones, partially crosslinked polyglycerin-modified silicones, linear or branched polyoxyethylene-modified organopolysiloxanes, linear or branched polyoxyethylene-polyoxypropylene-modified organopolysiloxanes, linear or branched polyoxyethylene-alkyl-co-modified organopolysiloxanes, linear or branched polyoxyethylene-polyoxypropylene-alkyl-co-modified organopolysiloxanes, linear or branched polyglycerin-modified organopolysiloxanes, linear or branched polyglycerin-alkyl-co-modified organopolysiloxanes, and a linear or branched pyrrolidone-modified organopolysiloxane. In these surfactants, a content of a hydrophilic polyoxyethylene group, a polyoxyethylene-polyoxypropylene group, or a polyglycerin residual group is preferably 10 to 70 mass% of the molecule. When the partially crosslinked polyether-modified silicone and the partially crosslinked polyglycerin-modified silicone is used, the crosslinked organopolysiloxane is preferably swelled, in a composition composed of the crosslinked organopolysiloxane and an oil agent being liquid at a room temperature, by containing the liquid oil agent having a weight equal to or larger than that of the crosslinked organopolysiloxane. For the liquid oil agent, liquid silicone, hydrocarbon oil, ester oil, natural animal or vegetable oil, semisynthetic oil, and fluorine-based oil, which are in the oil agent of any components, can be used. Examples thereof include: a low-viscous silicone having a kinematic viscosity of 0.65 to 100 mm²/s at 25°C; hydrocarbon oils such as a liquid paraffin, squalane, isododecane, and isohexadecane; glyceride oils such as triethylhexanoin; ester oils such as isotridecyl isononanoate; and natural animal or vegetable oils such as jojoba oil. Specific examples of the crosslinked organopolysiloxane include KSG-210, KSG-240, KSG-310, KSG-320, KSG-330, KSG-340, KSG-320Z, KSG-350Z, KSG-710, KSG-810, KSG-820, KSG-830, KSG-840, KSG-820Z, KSG-850Z, etc., which are all manufactured by Shin-Etsu Chemical Co., Ltd. Specific examples of the uncrosslinked organopolysiloxane include KF-6011, KF-6013, KF-6043, KF-6017, KF-6028, KF-6038, KF-6048, KF-6100, KF-6104, KF-6105, and KF-6106, which are all manufactured by Shin-Etsu Chemical Co., Ltd. In any cases, a blended amount of the surfactant is preferably 0.1 to 20 mass% of the entirety of the cosmetic. Depending on the purpose of the present invention, one or more kinds of crosslinked organopolysiloxanes, uncrosslinked organopolysiloxanes, or both may be selected as appropriate.

When the component (I) is blended, the contents is preferably in 0.01 to 15 mass% in the cosmetic.

### Component (J) : Crosslinked Organopolysiloxane

The crosslinked organopolysiloxane is not particularly limited as long as it is normally used for cosmetics, one of them may be used alone or two or more kinds thereof may be used in combination appropriately.

This crosslinked organopolysiloxane does not have a spherical shape, differently from the silicone spherical powder described in the above component (H).

Further, the component (J) is preferably a compound having no polyether nor polyglycerin structure in the molecular structure, differently from the above component (I) surfactant. Furthermore, the component (J) is preferably an elastomer having a structural viscosity caused by swelling with an oil agent. Specific examples thereof are defined by Japanese Labeling Name of Cosmetic Ingredient and include (dimethicone/vinyl dimethicone) crosspolymer, (dimethicone/phenyl vinyl dimethicone) crosspolymer, (vinyl dimethicone/lauryl dimethicone) crosspolymer, and (lauryl polydimethylsiloxy ethyl dimethicone/bisvinyl dimethicone) crosspolymer, etc. These are commercially available as a swelled product containing oil being liquid at a room temperature, and specific examples thereof include KSG-15, KSG-1510, KSG-16, KSG-1610, KSG-18A, KSG-19, KSG-41A, KSG-42A, KSG-43, KSG-44, KSG-042Z, KSG-045Z, KSG-048Z, etc., which are all manufactured by Shin-Etsu Chemical Co., Ltd.

When the component (J) is blended, the blending amount is preferably 0.01 to 30 mass% as a solid content in the cosmetic.

### Component (K): Coating Agent

As a coating agent, conventional film-forming agents can be used in a combination. Conventional coating agents is not particularly limited as long as it is a material that can be commonly blended in a cosmetic. Specific usable examples are: latexes such as polyvinyl alcohol, polyvinylpyrrolidone, polyvinyl acetate, and alkyl polyacrylate; cellulose derivatives such as dextrin, alkylcellulose, and nitrocellulose; siliconized polysaccharide compounds such as tri(trimethylsiloxy)silylpropylcarbamate pullulan; acryl-silicone-based grafted copolymers such as an (alkyl acrylate-dimethicone) copolymer; silicone resins such as trimethylsiloxylsilicic acid; silicone-based resins such as silicone-modified polynorbornene, a fluorine-modified silicone resin; fluororesins; aromatic hydrocarbon reins; polymer emulsion resins; terpene-based resins; polybutene; polyisoprene; alkyd resins; polyvinylpyrrolidone-modified polymers; rosin-modified resins, polyurethanes, etc.

Among these, silicone-based resins is particularly preferable, and those usable are, not limited to, tri(trimethylsiloxy)silylpropylcarbamate pullulan (the commercial product dissolved in a solvent are TSPL-30-D5 and ID, manufactured by Shin-Etsu Chemical Co., Ltd.), an (alkyl acrylate-dimethicone) copolymer (the commercial product dissolved in a solvent are KP-543, KP-545, KP-549, KP-550, KP-545L, etc., manufactured by Shin-Etsu Chemical Co., Ltd.), trimethylsiloxysilicic acid (the commercial product dissolved in a solvent are KF-7312J, X-21-5250, etc., manufactured by Shin-Etsu Chemical Co., Ltd.), silicone-modified polynorbornene (the commercial product dissolved in a solvent are NBN-30-ID, etc., manufactured by Shin-Etsu Chemical Co., Ltd.), an organosiloxane graft polyvinyl alcohol polymer, etc.

When the component (K) is blended, the blending amount is preferably 0.1 to 20 mass% in the cosmetic.

### Component (L): Other Additives

Examples of other additives include an oil-soluble gelling agent, a water-soluble thickener, an antiperspirant, antiseptic and bactericide, a perfume, a salt, an antioxidant, a pH regulator, a chelating agent, a refreshing agent, an anti-inflammatory agent, a skin-beautifying component (such as a whitening agent, a cell activator, a rough skin improver, a blood circulation promoter, a skin astringent, and an antiseborrheic agent), vitamins, amino acids, a nucleic acid, a hormone, a inclusion compound, etc. One of these components (L) may be used alone or two or more kinds thereof may be used in combination appropriately. When the component (L) is blended, the blending amount is preferably 0.1 to 20 mass% in the cosmetic.

### ·Oil-Soluble Gelation Agent

Examples of the oil-soluble gelation agent include: metal soaps such as aluminum stearate, magnesium stearate, and zinc myristate; amino acid derivatives such as N-lauroyl-L-glutamic acid and α,γ-di-n-butylamine; dextrin fatty acid esters such as dextrin palmitate ester, dextrin stearate ester, and dextrin 2-ethylhexanoate palmitate ester; sucrose fatty acid esters such as sucrose palmitate ester and sucrose stearate ester; fructo-oligosaccharide fatty acid esters such as fructo-oligosaccharide stearate ester and fructo-oligosaccharide 2-ethylhexanoate ester; sorbitol benzylidene derivatives such as monobenzylidene sorbitol and dibenzylidene sorbitol; and organic-modified clay minerals such as disteardimonium hectorite, stearalkonium hectorite, and hectorite; etc.

### ·Water-Soluble Thickener

Examples of the water-soluble thickener include: vegetable polymers such as gum arabic, tragacanth, galactan, carob gum, guar gum, karaya gum, carrageenan, pectin, agar, quince seed (marmelo), starch (rice, corn, potato, wheat, etc.), algae colloid, trant gum, and locust bean gum; microorganic polymers such as xanthan gum, dextran, succinoglucan, and pullulan; animal polymers such as collagen, casein, albumin, and gelatin; starch-based polymers such as carboxymethylstarch and methylhydroxypropylstarch; cellulose-based polymers such as methylcellulose, ethylcellulose, methylhydroxypropylcellulose, carboxymethylcellulose, hydroxymethylcellulose, hydroxypropylcellulose, nitrocellulose, sodium cellulose sulfate, sodium carboxymethylcellulose, crystalline cellulose, a cationized cellulose, and a cellulose; alginic acid-based polymers such as sodium alginate and propylene glycol alginate ester; vinylic polymers such as polyvinyl methyl ether and a carboxyvinyl polymer; polyoxyethylene-based polymers; polyoxyethylene-polyoxypropylene copolymers-based polymers; acrylic polymers such as sodium polyacrylate, polyethyl acrylate, polyacrylamide, and an acryloyl dimethyl taurine salt copolymer; other synthetic water-soluble polymers such as polyethyleneimine and cation polymer; and inorganic water-soluble polymers such as bentonite, aluminum magnesium silicate, montmorillonite, beidellite, nontronite, saponite, hectorite, and silicic anhydride; etc.

Among these, preferably usable are one or more water-soluble thickeners of one kind or combination of two or more kinds selected from the group consisting of vegetable polymers, microorganic polymers, animal polymers, starch-based polymers, cellulose-based polymers, alginic acid-based polymers, polyoxyethylene-polyoxypropylene copolymer-based polymers, acrylic polymers, and inorganic water-soluble polymers.

### ·Antiperspirant

Examples of the antiperspirant include: aluminum hydroxyhalides such as chlorohydroxyaluminum and allantoin chlorohydroxyaluminum; aluminum halides such as aluminum chloride; allantoin aluminum salts, tannic acid, persimmon tannin, aluminum potassium sulfate, zinc oxide, zinc paraphenolsulfonate, burnt alum, aluminum zirconium tetrachlorohydrate, aluminum zirconium trichlorohydrex glycine, etc. In particular, preferable antiperspirants exhibiting a high effect are aluminum hydroxyhalides, aluminum halides, and a complex or mixture thereof with zirconyl oxyhalide and zirconyl hydroxyhalide (for example, aluminum zirconium tetrachlorohydrate, aluminum zirconium trichlorohydrex glycine, etc.).

### ·Antiseptics and Bactericide

Examples of antiseptics and bactericides include a paraoxybenzoic acid alkyl ester, benzoic acid, sodium benzoate, sorbic acid, potassium sorbate, phenoxyethanol, imidazolidinylurea, salicylic acid, isopropylmethylphenol, carbolic acid, parachlorometacresol, hexachlorophene, benzalkonium chloride, chlorhexidine chloride, trichlorocarbanilide, iodopropynyl butylcarbamate, polylysine, a photosensitizer, silver, a vegetable extract, etc.

### ·Perfume

The perfumes include natural perfumes and synthetic perfumes. Examples of the natural perfumes include: vegetable perfumes isolated from flowers, leaves, wood, pericarps, etc.; and animal perfumes such as musk and civet. Examples of the synthetic perfumes include: hydrocarbons such as monoterpene; alcohols such as aliphatic alcohols and aromatic alcohols; aldehydes such as terpene aldehydes and aromatic aldehydes; ketones such as alicyclic ketones; esters such as terpene-based esters; lactones; phenols; oxides; nitrogen-containing compounds; acetals; etc.

### ·Salt

Examples of the salts include inorganic salts, organic acid salts, amine salts, and amino acid salts. Examples of the inorganic salts include sodium salts, potassium salts, magnesium salts, calcium salts, aluminum salts, zirconium salts, and zinc salts of inorganic acids such as hydrochloric acid, sulfuric acid, carbonic acid, and nitric acid. Examples of the organic acid salts include salts of organic acids such as acetic acid, dehydroacetic acid, citric acid, malic acid, succinic acid, ascorbic acid, and stearic acid. Examples of the amine salts and amino acid salts include: salts of amines such as triethanolamine; and salts of amino acids such as glutamic acid. In addition, it is possible to use salts of hyaluronic acid, chondroitin sulfuric acid, and neutralized salts of an acid and a base used in formulation, etc.

### ·Antioxidant

The antioxidants are not particularly limited, and examples thereof include carotinoide, ascorbic acid and a salt thereof, ascorbyl stearate, tocophenol, tocophenyl acetate, tocopherol, p-t-butylphenol, butylhydroxyanisole, dibutylhydroxytoluene, phytic acid, ferulic acid, thiotaurine, hypotaurine, a sulfite salt, erythorbic acid and a salt thereof, chlorogenic acid, epicatechin, epigallocatechin, epigallocatechin gallate, apigenin, kaempferol, myricetin, quercetin, etc.

### ·pH Regulator

Examples of the pH regulator include lactic acid, citric acid, glycolic acid, succinic acid, tartaric acid, dl-malic acid, potassium carbonate, sodium hydrogen carbonate, ammonium hydrogen carbonate, etc.

### ·Chelating Agent

Examples of the chelating agent include alanine, sodium edetate salt, sodium polyphosphate, sodium metaphosphate, phosphoric acid, etc.

### ·Refreshing Agent

Examples of the refreshing agent include L-menthol, camphor, menthyl lactate, etc.

### ·Anti-Inflammatory Agent

Examples of the anti-inflammatory agent include allantoin, glycyrrhizic acid and a salt thereof, glycyrrhetinic acid and stearyl glycyrrhetinate, tranexamic acid, azulene, etc.

### ·Skin-Beautifying Component

Examples of the skin-beautifying component include: whitening agents such as placenta extract, arbutin, glutathione, and Saxifraga extract; cell activators such as royal jelly, a photosensitizer, a cholesterol derivative, and calf blood extract; rough skin improvers; blood circulation promoters such as nonylwallenylamide, nicotinic acid benzyl ester, nicotinic acid β-butoxyethyl ester, capsaicin, zingerone, cantharides tincture, ichthammol, caffeine, tannic acid, α-borneol, tocopheryl nicotinate, inositol hexanicotinate, cyclandelate, cinnarizine, tolazoline, acetylcholine, verapamil, cepharanthine, and γ-oryzanol; skin astringents; antiseborrheic agent such as sulfur and thiantrol; etc.

### ·Vitamin

Examples of the vitamins include: vitamin A such as vitamin A oil, retinol, retinyl acetate, and retinyl palmitate; vitamin B such as vitamin B₂ such as riboflavin, riboflavin butyrate, and flavin adenine nucleotide, vitamin B₆ such as pyridoxine hydrochloride salt, pyridoxine dioctanoate, and pyridoxine tripalmitate, vitamin B₁₂ and a derivative thereof, and vitamin B₁₅ and a derivative thereof; vitamin C such as L-ascorbic acid, L-ascorbic acid dipalmitate ester, L-ascorbic acid-2-sodium sulfate, and L-ascorbic acid dipotassium phosphate diester; vitamin D such as ergocalciferol and cholecalciferol; vitamin E such as α-tocopherol, β-tocopherol, γ-tocopherol, dl-α-tocopheryl acetate, dl-α-tocopheryl nicotinate, and dl-α-tocopheryl succinate; nicotinic acids such as nicotinic acid, benzyl nicotinate, and nicotinamide; vitamin H; vitamin P; pantothenic acids such as calcium pantothenate, D-pantothenyl alcohol, pantothenyl ethyl ether, and acetylpantothenyl ethyl ether; biotin; etc.

### ·Amino acid

Examples of the amino acids include glycine, valine, leucine, isoleucine, serine, threonine, phenylalanine, arginine, lysine, aspartic acid, glutamic acid, cystine, cysteine, methionine, tryptophan, etc.

### ·Nucleic Acid

Examples of the nucleic acid include a deoxyribonucleic acid, etc.

### ·Hormone

Examples of the hormone include estradiol, ethenylestradiol, etc.

### ·Inclusion Compound

Examples of the inclusion compound include cyclodextrin, etc.

The cosmetic in the present invention is not particularly limited, and can be applied for various products such as, for example, cosmetic essence, milky lotion, cream, hair care, foundation, cosmetic base, sunscreen, concealer, cheek color, lipstick, gloss, balm, mascara, eye shadow, eye liner, body make-up, deodorant agent, and a cosmetic for nail. Among these, make-up cosmetics such as milky lotion, cream, hair care, and foundation, and a cosmetic with an imparted sunscreen effect are particularly preferable. As forms of the inventive cosmetic, various forms such as liquid, cream, solid, paste, gel, mousse, souffle, cray, powder, and stick can be selected.

### EXAMPLE

Hereinafter, the present invention will be described in more detail with reference to Manufacturing Example, Examples, Manufacturing Comparative Example, and Comparative Example, but the present invention is not limited to the following Manufacturing Example, Examples. "%" in the composition is mass% unless otherwise specified. Note that, hydrophobic cellulose nanofibers and their dispersions are referred to as Manufacturing Examples and Manufacturing Comparative Examples, and cosmetics are referred to as Examples and Comparative Examples.

### [Manufacturing Example 1]

### <Manufacturing of KF-96A-6cs dispersion of phosphite ester group-containing hydrophobic cellulose nanofiber>

(Step 1) 100g (1.0g in weight of cellulose nanofiber) of 1.0 mass% aqueous dispersion of phosphite ester group-containing cellulose nanofiber (manufactured by Daio Paper Corporation, average fiber diameter: 5 nm, light transmittance: 92%) and 400g of N-methyl-2-pyrrolidone (NMP) were weighed into a 2L descup and mixed using a high-speed dispersion mixer (manufactured by Silverson Machines Ltd.) to obtain a water-NMP mixed dispersion of phosphite ester group-containing cellulose nanofiber. Next, the mixed dispersion was sufficiently dehydrated by a reduced pressure-distilling off to obtain a NMP dispersion of the phosphite ester group-containing cellulose nanofiber. At this time, the amount of water contained in the dispersion was 300 ppm.

(Step 2) 0.3 g of di-n-butyltin dilaurate was added as a urethanization catalyst to the NMP dispersion of phosphite ester group-containing cellulose nanofiber obtained in Step 1, and the mixture was maintained at 80°C. 14.0 g (charged weight of isocyanate group-containing organopolysiloxane/charged weight of cellulose nanofibers = 14.0) of isocyanate group-containing organopolysiloxane represented by the following formula (5) was added dropwise to the dispersion and the reaction was continued by heating at 80°C for 3 hours. After confirming the disappearance of the band derived from isocyanate groups by FT-IR, unreacted isocyanate groups were deactivated by adding 1.0 g of ethanol, to obtain an NMP dispersion of the phosphite ester group-containing hydrophobic cellulose nanofiber.

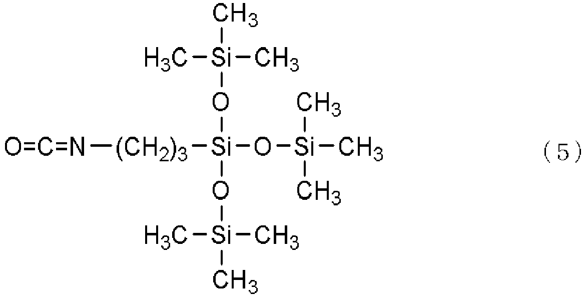

(Step 3) By repeating three times of operation of adding 2-propanol to the NMP dispersion of the phosphite ester group-containing hydrophobic cellulose nanofiber obtained in Step 2 and then recovering it by filtration, gel that was phosphite ester group-containing hydrophobic cellulose nanofiber swollen with the 2-propanol was obtained. 100 g of KF-96A-6cs was added to the obtained gel, and the mixture was homogenized by mixing using a high-speed dispersion mixer (manufactured by Silverson Machines Ltd.). The remaining 2-propanol was sufficiently removed by distillation under reduced pressure, to obtain a KF-96A-6cs dispersion of the phosphite ester group-containing hydrophobic cellulose nanofiber. The physical properties of the obtained dispersion are shown in Table 1.

### [Manufacturing Example 2]

### <Manufacturing of methyl trimethicone dispersion of phosphate ester group-containing cellulose nanofiber>

"100g (1.0g in weight of cellulose nanofibers) of 1.0 mass% aqueous dispersion of a phosphite ester group-containing cellulose nanofiber (manufactured by Daio Paper Corporation, average fiber diameter: 5 nm, light transmittance: 92%)" of Manufacturing Example 1 was changed to "50g (1.0g in weight of cellulose nanofibers) of 2.0 mass% aqueous dispersion of a phosphate ester group-containing cellulose nanofiber (manufactured by Oji Paper Co., Ltd., average fiber diameter: 4 nm, light transmittance: 95%)", and "KF-96A-6cs" was changed to "methyl trimethicone", Other than the above, the same steps of the Manufacturing Example 1 were repeated to obtain a methyl trimethicone dispersion of phosphate ester group-containing hydrophobic cellulose nanofiber. The physical properties of the obtained dispersion are shown in Table 1.

### [Manufacturing Example 3]

### <Manufacturing of methyl trimethicone dispersion of carboxyl group-containing cellulose nanofiber>

"100g (1.0g in weight of cellulose nanofibers) of 1.0 mass% aqueous dispersion of a phosphite ester group-containing cellulose nanofiber (manufactured by Daio Paper Corporation, average fiber diameter: 5 nm, light transmittance: 92%)" of Manufacturing Example 1 was changed to "50g (1.0g in weight of cellulose nanofibers) of 2.0 mass% aqueous dispersion of a carboxyl group-containing cellulose nanofiber (manufactured by Dai-ichi Kogyo Seiyaku Co., Ltd., average fiber diameter: 3 nm, light transmittance: 95%)", and "KF-96A-6cs" was changed to "methyl trimethicone". Other than the above, the same steps of the Manufacturing Example 1 were repeated to obtain the methyl trimethicone dispersion of the carboxyl group-containing hydrophobic cellulose nanofiber. The physical properties of the obtained dispersion are shown in Table 1.

### [Manufacturing Example 4]

### <Manufacturing of decamethylcyclopentasiloxane dispersion of phosphite ester group-containing hydrophobic cellulose nanofiber>

"KF-96A-6cs" of Manufacturing Example 1 was changed to "decamethylcyclopentasiloxane". Other than the above, the same steps of the Manufacturing Example 1 were repeated to obtain the decamethylcyclopentasiloxane dispersion of the phosphite ester group-containing hydrophobic cellulose nanofiber. The physical properties of the obtained dispersion are shown in Table 1.

### [Manufacturing Example 5]

### <Manufacturing of methyl trimethicone dispersion of phosphite ester group-containing hydrophobic cellulose nanofiber>

"KF-96A-6cs" of Manufacturing Example 1 was changed to "methyl trimethicone". Other than the above, the same steps of the Manufacturing Example 1 were repeated to obtain the methyl trimethicone dispersion of the phosphite ester group-containing hydrophobic cellulose nanofiber. The physical properties of the obtained dispersion are shown in Table 1.

### [Manufacturing Example 6]

### <Manufacturing of methyl trimethicone dispersion of phosphite ester group-containing hydrophobic cellulose nanofiber>

"KF-96A-6cs" of Manufacturing Example 1 was changed to "methyl trimethicone", "13.8 g of isocyanate group-containing organopolysiloxane represented by the formula (5)" was changed to "14.9 g of isocyanate group-containing organopolysiloxane represented by the formula (6)", and the urethanization catalyst was changed from "0.3 g of di-n-butyltin dilaurate" to "6.0 g of triethylamine". Other than the above, the same steps of the Manufacturing Example 1 were repeated to obtain the methyl trimethicone dispersion of the phosphite ester group-containing hydrophobic cellulose nanofiber. The physical properties of the obtained dispersion are shown in Table 1.

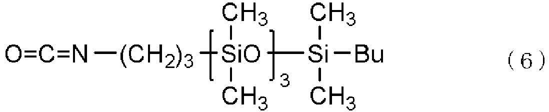

### [Manufacturing Example 7]

### <Manufacturing of toluene dispersion of phosphite ester group-containing hydrophobic cellulose nanofiber>

"KF-96A-6cs" of Manufacturing Example 1 was changed to "toluene", and "13.8 g of isocyanate group-containing organopolysiloxane represented by the formula (5)" was changed to "14.9 g of isocyanate group-containing organopolysiloxane represented by the formula (6)". Other than the above, the same steps of the Manufacturing Example 1 were repeated to obtain a toluene dispersion of the phosphite ester group-containing hydrophobic cellulose nanofiber. The physical properties of the obtained dispersion are shown in Table 1.

### [Manufacturing Example 8]

### <Manufacturing of 1-butanol dispersion of phosphite ester group-containing hydrophobic cellulose nanofiber>

"KF-96A-6cs" of Manufacturing Example 1 was changed to "1-butanol", and "N-methyl-2-pyrrolidone" was changed to "N,N-dimethylformamide". Other than the above, the same steps of the Manufacturing Example 1 were repeated to obtain a 1-butanol dispersion of the phosphite ester group-containing hydrophobic cellulose nanofiber. The physical properties of the obtained dispersion are shown in Table 1.

### [Manufacturing Example 9]

### <Manufacturing of methyl trimethicone dispersion of phosphite ester group-containing hydrophobic cellulose nanofiber>

"KF-96A-6cs" of Manufacturing Example 1 was changed to "methyl trimethicone", the charged weight of isocyanate group-containing organopolysiloxane/charged weight of cellulose nanofibers was changed to 4.5, and the urethanization catalyst was changed from "0.3 g of di-n-butyltin dilaurate" to "6.0 g of triethylamine". Other than the above, the same steps of the Manufacturing Example 1 were repeated to obtain a methyl trimethicone dispersion of the phosphite ester group-containing hydrophobic cellulose nanofiber. The physical properties of the obtained dispersion are shown in Table 1.

### [Manufacturing Example 10]

### <Manufacturing of methyl trimethicone dispersion of phosphite ester group-containing hydrophobic cellulose nanofiber>

"KF-96A-6cs" of Manufacturing Example 1 was changed to "methyl trimethicone", the charged weight of isocyanate group-containing organopolysiloxane/charged weight of cellulose nanofibers was changed to 28.0, and the urethanization catalyst was changed from "0.3 g of di-n-butyltin dilaurate" to "6.0 g of triethylamine". Other than the above, the same steps of the Manufacturing Example 1 were repeated to obtain a methyl trimethicone dispersion of the phosphite ester group-containing hydrophobic cellulose nanofiber. The physical properties of the obtained dispersion are shown in Table 1.

### [Comparative Manufacturing Example 1]

### <Manufacturing of mixture of phosphite ester group-containing hydrophobic cellulose nanofiber and methyl trimethicone>

"KF-96A-6cs" of Manufacturing Example 1 was changed to "methyl trimethicone", and "isocyanate group-containing organopolysiloxane represented by the formula (5)" was not used. Other than the above, the same steps of the Manufacturing Example 1 were repeated to obtain a mixture of phosphite ester group-containing hydrophobic cellulose nanofiber and methyl trimethicone. The physical properties of the obtained mixture are shown in Table 1.

### [Comparative Manufacturing Example 2]

### <Manufacturing of mixture of phosphite ester group-containing hydrophobic cellulose nanofiber and methyl trimethicone>

"KF-96A-6cs" of Manufacturing Example 1 was changed to "methyl trimethicone", and "isocyanate group-containing organopolysiloxane represented by the formula (5)" was changed to "butylisocyanate". Other than the above, the same steps of the Manufacturing Example 1 were repeated to obtain a mixture of phosphite ester group-containing hydrophobic cellulose nanofiber and methyl trimethicone. The physical properties of the obtained mixture are shown in Table 1.

### [Comparative Manufacturing Example 3]

### <Manufacturing of mixture of phosphite ester group-containing hydrophobic cellulose nanofiber and methyl trimethicone>

"KF-96A-6cs" of Manufacturing Example 1 was changed to "methyl trimethicone", and "isocyanate group-containing organopolysiloxane represented by the formula (5)" was changed to "dodecyl isocyanate". Other than the above, the same steps of the Manufacturing Example 1 were repeated to obtain a mixture of phosphite ester group-containing hydrophobic cellulose nanofiber and methyl trimethicone. The physical properties of the obtained mixture are shown in Table 1.

### <Dispersibility>

The cellulose nanofiber dispersions obtained in Manufacturing Examples 1 to 10 and the cellulose nanofiber mixtures obtained in Comparative Manufacturing Examples 1 to 3 were visually checked for the presence or absence of sedimentation of cellulose nanofibers. The case where sedimentation was not confirmed was evaluated as "Good," and the case where sedimentation was confirmed was evaluated as "Poor." The results are listed in Table 1.

### <Calculation of average fiber diameter of hydrophobic cellulose nanofibers>

The cellulose nanofiber dispersions obtained in Manufacturing Examples 1 to 10 were prepared to have a solid content concentration of 0.01 mass%, dropped onto a copper grid, dried, and used as an observation sample for observation using a transmission electron microscope. Three or more images in which the fibers do not overlap were observed, and the diameters of 20 fibers per image were calculated. The average of them was determined to be the average fiber diameter. The results are shown in Table 1.

### <Light transmittance>

The cellulose nanofiber dispersions obtained in Manufacturing Examples 1 to 10 were diluted to a concentration of 0.2 mass%, filled in a square cell having an optical path length of 10 mm. Using an ultraviolet-visible spectrophotometer (UV-1800, Shimadzu Corporation), the light transmittance at a wavelength of 600 nm was measured. The results are listed in Table 1.

### <Viscosity measurement>

For the cellulose nanofiber dispersions obtained in Manufacturing Examples 1 to 10, the viscosity was measured using a B-type rotational viscometer (Brookfield) after 1 minute of the rotor rotating at 6 rpm. The results are listed in Table 1.

### <Film property>

The cellulose nanofiber dispersions obtained in Manufacturing Examples 2 to 10 and the cellulose nanofiber mixtures obtained in Comparative Manufacturing Examples 1 to 3 were dried in an oven at 105°C for 3 hours. The state in which the solvent had evaporated by drying was visually observed and evaluated based on the following criteria. The results are listed in Table 1. Note that, Manufacturing Example 1 uses a non-volatile oil agent and thus is excluded from the evaluation of film properties.
Excellent: A uniform film with a colorless and transparent appearance is obtained.
Good: A uniform film with a white or cloudy appearance is obtained.
Poor: No film is obtained (powdery), a film with uneven appearance (spotted) is obtained.

**[Table 1]**

| | Manufacturing Example | | | | | | |
|---|---|---|---|---|---|---|---|
| | 1 | 2 | 3 | 4 | 5 | 6 | 7 |
| Anionic functional group in cellulose nanofiber | Phosphite ester group | Phosphate ester group | Carboxyl group | Phosphite ester group | Phosphite ester group | Phosphite ester group | Phosphite ester group |
| Organopolysiloxane | Formula (I) | Formula (I) | Formula (I) | Formula (I) | Formula (I) | Formula (II) | Formula (II) |
| Dispersion medium | KF-96A-6cs | TMF-1.5 | TMF-1.5 | KF-995 | TMF-1.5 | TMF-1.5 | Toluene |
| Ratio of Organopolysiloxane *1 | 14.0 | 14.0 | 14.0 | 14.0 | 14.0 | 14.0 | 14.0 |
| Concentration of cellulose nanofiber in dispersion / % | - | 0.83 | 0.85 | 0.91 | 0.89 | 0.85 | 0.86 |
| Dispersibility | Good | Good | Good | Good | Good | Good | Good |
| Fiber diameter (nm) | 4.0 | 4.5 | 6.0 | 3.9 | 4.0 | 6.2 | 5.8 |
| Transmittance / % | 95 | 94 | 90 | 95 | 95 | 87 | 90 |
| viscosity / mPa·s | 32 | 12 | 18 | 26 | 15 | 16 | 8 |
| Film property | - | Excellent | Excellent | Excellent | Excellent | Excellent | Excellent |

| | Manufacturing Example | | | Comparative Manufacturing Example | | | |
|---|---|---|---|---|---|---|---|
| | 8 | 9 | 10 | 1 | 2 | 3 | |
| Anionic functional group in cellulose nanofiber | Phosphite ester group | Phosphite ester group | Phosphite ester group | Phosphite ester group | Phosphite ester group | Phosphite ester group | |
| Organopolysiloxane | Formula (I) | Formula (I) | Formula (I) | - | Butyl Isocyanate | Dodecyl Isocyanate | |
| Dispersion medium | 1-butanol | TMF-1.5 | TMF-1.5 | TMF-1.5 | TMF-1.5 | TMF-1.5 | |
| Ratio of Organopolysiloxane *1 | 14.0 | 4.5 | 28.0 | - | 4.5 | 4.5 | |
| Concentration of cellulose nanofiber in dispersion / % | 0.85 | 0.91 | 0.93 | - | - | - | |
| Dispersibility | Good | Good | Good | Poor | Poor | Poor | |
| Fiber diameter (nm) | 6.5 | 8.0 | 6.5 | - | - | - | |
| Transmittance / % | 89 | 82 | 88 | - | - | - | |
| Viscosity / mPa·s | 15 | 8 | 20 | - | - | - | |
| Film property | Excellent | Good | Excellent | Poor | Poor | Poor | |

| | | | | | | | |
|---|---|---|---|---|---|---|---|
| *1: Ratio of organopolysiloxane: Mass of mixed organopolysiloxane / Mass of cellulose nanofibers KF-96A-6cs: Dimethicone (with a kinematic viscosity at 25°C of 6 mm²/s, manufactured by Shin-Etsu Chemical Co., Ltd.) TMF-1.5: Methyl trimethicone (manufactured by Shin-Etsu Chemical Co., Ltd.) KF-995: Decamethylcyclopentasiloxane (manufactured by Shin-Etsu Chemical Co., Ltd.) | | | | | | | |

As shown in Manufacturing Examples 1 to 10 in Table 1, hydrophobic cellulose nanofiber dispersions were obtained, and each of them has a transmittance of 80% or more and their average fiber diameter of the cellulose nanofibers were all 300 nm or less. As shown in Manufacturing Examples 4, 5, and 8, the result shows that high dispersibility is achieved even when the properties of the dispersion medium are different. In addition, as shown in Manufacturing Examples 2, 3, and 5, the result shows that high dispersibility is achieved even when the types of anionic functional groups are different. On the other hand, in Comparative Manufacturing Example 1, dispersion in methyl trimethicone was attempted without blocking the hydroxy groups, but aggregates of cellulose nanofibers were formed and precipitates were observed. Further, in Comparative Manufacturing Examples 2 and 3, blockade with an alkyl isocyanate containing no siloxane component was attempted, but precipitates were observed in all cases, and the dispersibility did not improve. These indicates that the inventive hydrophobic cellulose nanofibers block hydroxy groups with a siloxane structure containing isocyanate groups and contribute to hydrophobization and dispersibility in specific oils such as alcohol, silicone oil, and hydrocarbon oil.

### (1) Characteristic evaluation

For Examples 1 to 12 and Comparative Examples 1 to 6 below, feeling on use (non-stickiness), refreshing feeling (freshness), applicability (good spreadability), and stability over time (The condition after storage at 50°C for 1 month) of the cosmetics was evaluated according to the evaluation criteria shown in Table 2. Based on the average value of 10 people, the results were judged according to the following criteria. The results are shown in Tables 3 to 5.

**[Table 2]**

| Evaluation criteria | | | | |
|---|---|---|---|---|
| Item | Feeling on use | Refreshing feeling | Coatablity | Stability over time |
| 5 points | Good | Good | Good | Good |
| 4 points | Somewhat good | Somewhat good | Somewhat good | Somewhat good |
| 3 points | Average | Average | Average | Average |
| 2 points | Somewhat Bad | Somewhat Bad | Somewhat Bad | Somewhat Bad |
| 1 point | Bad | Bad | Bad | Bad |

### Judgment criteria

Excellent: Average score is 4.5 points or more
Good: Average score is 3.5 points or more and less than 4.5 points
Fair: Average score is 2.5 points or more and less than 3.5 points
Poor: Average score is 1.5 points or more and less than 2.5 points
Very poor: Average score is less than 1.5 points

### <Examples>

W/O emulsions were prepared according to formulation shown in Table 3 below.

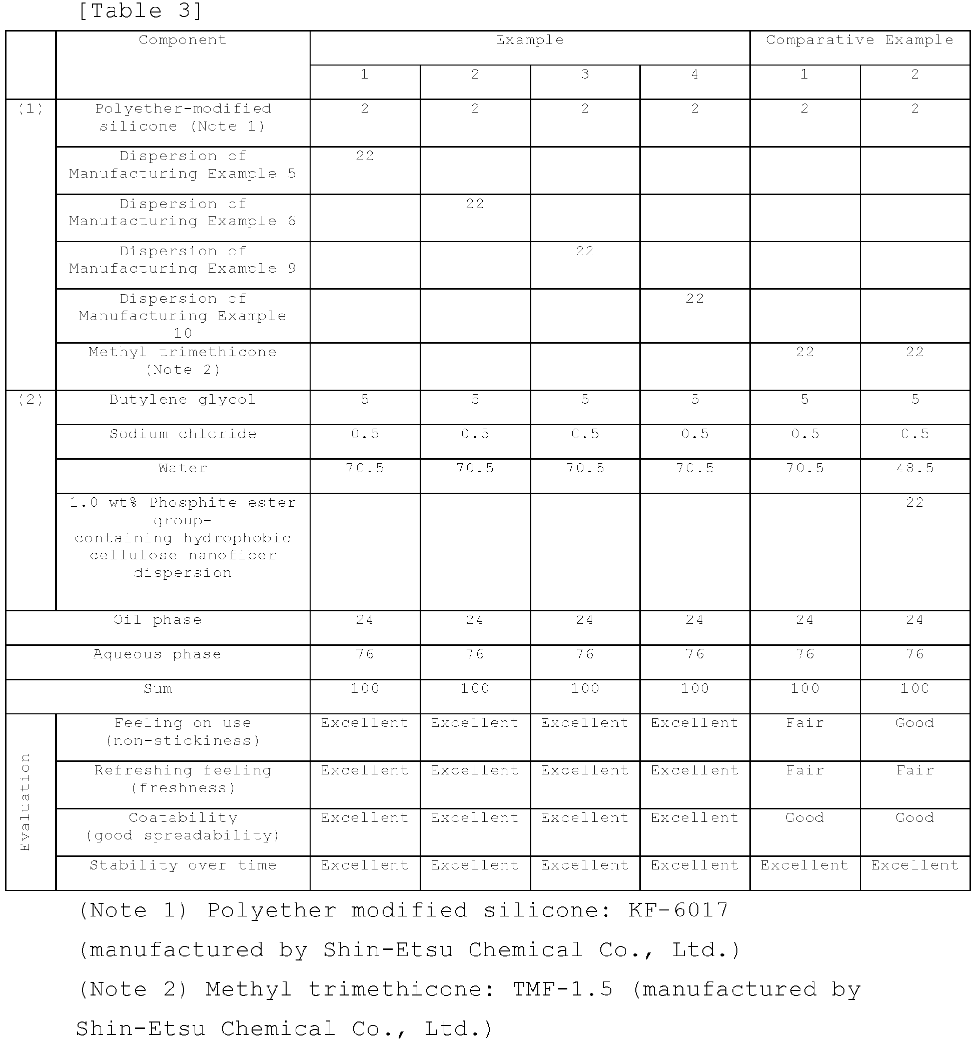

Note that, the blended amounts are the amount of the blended products (the same applies below).

### <Preparation of cosmetics>

A: Component (1) was mixed uniformly.
B: Component (2) was mixed uniformly.
C: B was added to A and emulsified to obtain a W/O emulsion.

From the results shown in Table 3 above, the W/O emulsions of Examples 1 to 4 were found to be excellent in "Feeling on use (non-stickiness)", "Refreshing feeling (freshness)", "Coatability (good spreadability)", and "Stability over time (after 1 month storage at 50°C)". On the other hand, in Comparative Example 1 in which the cellulose nanofiber dispersion is not added, the feel derived from cellulose nanofibers is not imparted, and in consequence "Feeling on use", "Refreshing feeling", and "Coatability" are impaired. Further, in Comparative Example 2 in which the cellulose nanofiber is dispersed in water, the cellulose nanofibers were present in the internal phase because the emulsion was a water-in-oil type and does not affect the feeling during application, and in consequence the feeling on use and the refreshing feeling were impaired.

W/O emulsion having the composition shown in Table 4 below was prepared.

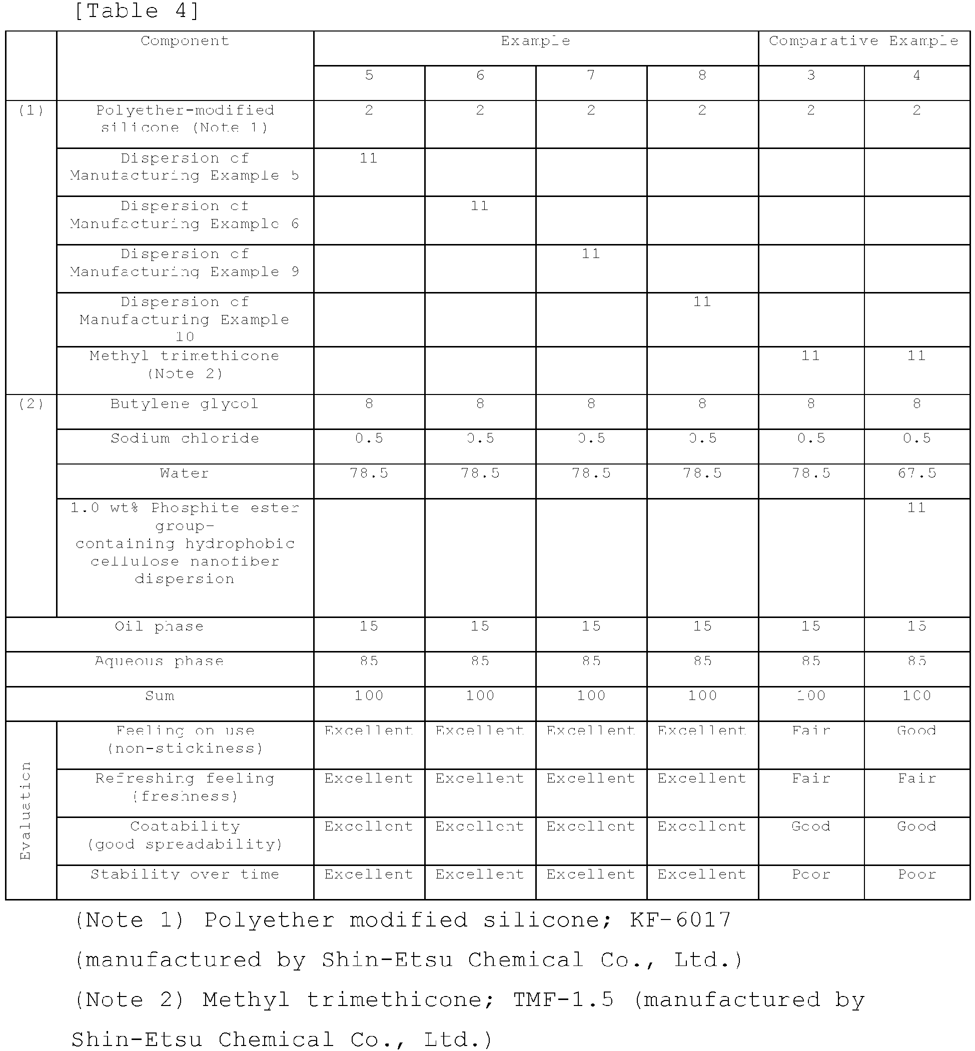

Note that, the blended amounts are the amount of the blended products (The same applies below).

### <Preparation of cosmetics>

A: Component (1) was mixed uniformly.
B: Component (2) was mixed uniformly.
C: B was added to A and emulsified to obtain a W/O emulsion.

From the results shown in Table 4 above, the W/O emulsions of Examples 5 to 8 were found to be excellent in "Feeling on use (non-stickiness)", "Refreshing feeling (freshness)", "Coatability (good spreadability)", and "Stability over time (after 1 month storage at 50°C)". On the other hand, in Comparative Example 3 in which the cellulose nanofiber dispersion is not added, the feel derived from cellulose nanofibers is not imparted, and in consequence "Feeling on use", "Refreshing feeling", and "Coatability" are impaired. In addition, the increase in the aqueous phase deteriorated the over time stability. This is because cellulose nanofibers serve as a thickener for the oil phase, which has the effect of increasing the stability of the entire system. Further, in Comparative Example 4 in which the cellulose nanofiber is dispersed in water, the cellulose nanofibers were present in the internal phase because the emulsion was a water-in-oil type and does not affect the feeling during application, and in consequence the feeling on use and the refreshing feeling were impaired. Furthermore, when cellulose nanofibers were used in the internal phase, the over time stability deteriorated because they did not contribute to stabilizing the entire system.

Emulsified cream foundations having the composition shown in Table 5 below was prepared.

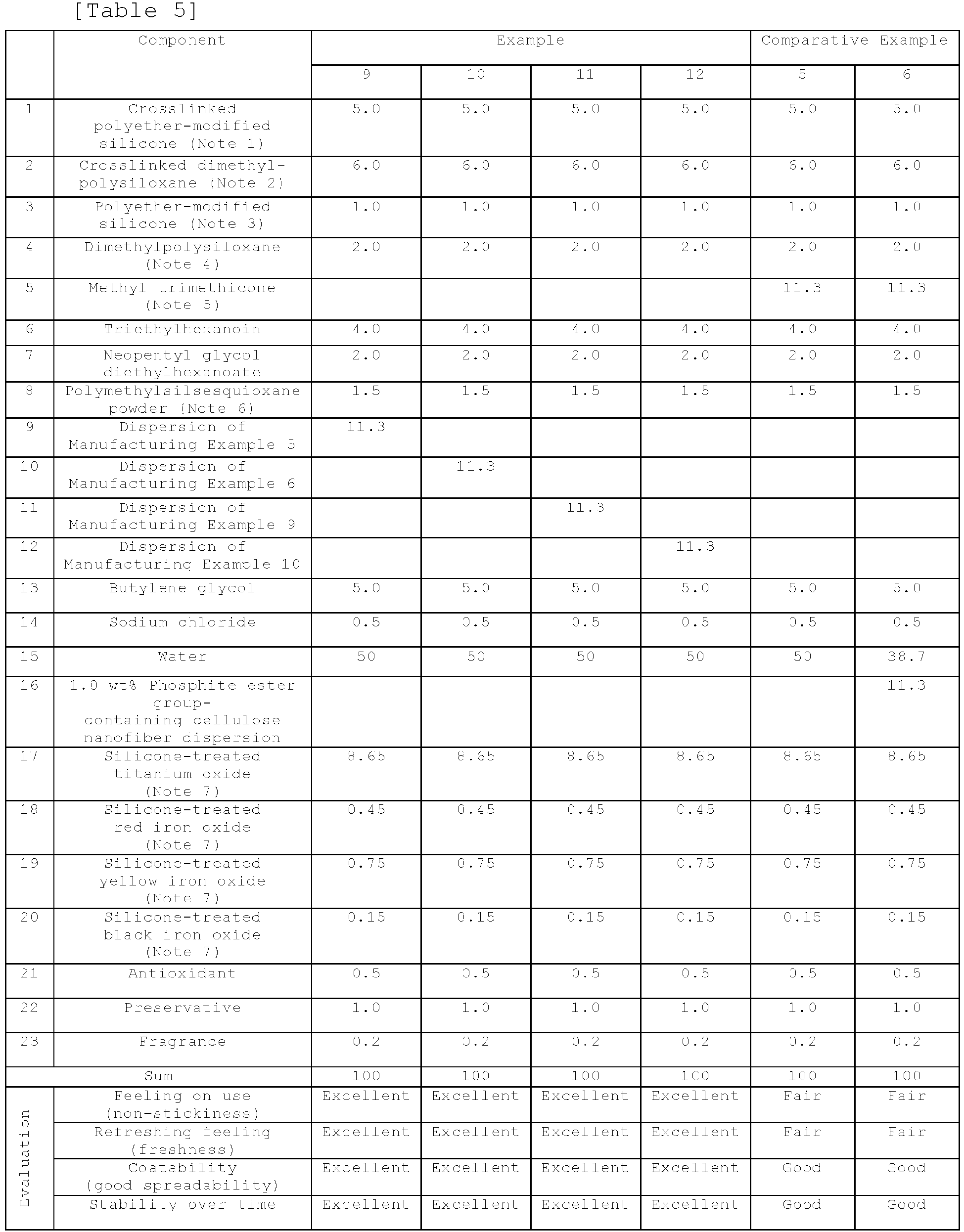

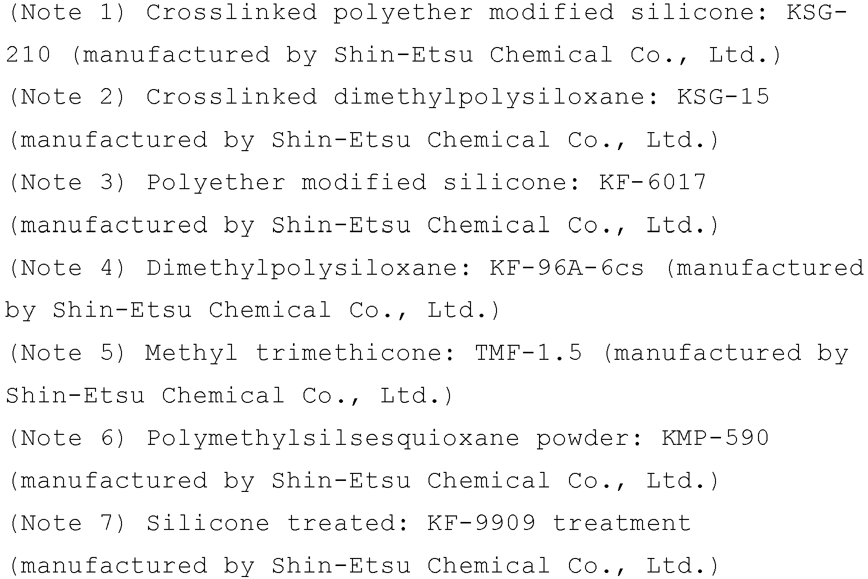

### <Preparation of cosmetics>

Components (1) to (4), a portion of (5), (6) to (12), and (21) to (22) were stirred and mixed to be uniform. This mixture was gently added with components (13) to (14) and (16), which were separately uniformly dissolved in component (15), and then emulsified. This emulsion was added with components (17) to (20), the remainder of (5), and (23) and then mixed. This mixture was filled into a predetermined container to prepare an emulsified cream foundation.

From the results shown in Table 5 above, the emulsified cream foundations of Examples 9 to 12 were found to be excellent in "Feeling on use (non-stickiness)", "Refreshing feeling (freshness)", "Coatability (good spreadability)", and "Stability over time (after 1 month storage at 50°C)". On the other hand, in Comparative Example 5 in which the cellulose nanofiber dispersion is not added, the feel derived from cellulose nanofibers is not imparted, and in consequence "Feeling on use", "Refreshing feeling", and "Coatability" are impaired. Further, in Comparative Example 6 in which the cellulose nanofiber is dispersed in water, the cellulose nanofibers were present in the internal phase because the emulsion was a water-in-oil type and does not affect the feeling during application, and in consequence the feeling on use and the refreshing feeling were impaired.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A hydrophobic cellulose nanofiber comprising:
(A) a cellulose nanofiber; and
(B) isocyanate group-containing organopolysiloxane, wherein the isocyanate group-containing organopolysiloxane is bonded to a hydroxy group of the cellulose nanofiber through a urethane bond.

2. The hydrophobic cellulose nanofiber according to claim 1, wherein
the cellulose nanofiber (A) further comprises an anionic functional group.

3. The hydrophobic cellulose nanofiber according to claim 2, wherein
0.2 mass% aqueous dispersion of the cellulose nanofiber having the anionic functional group has a 600 nm light transmittance of 80% or more and the cellulose nanofiber having the anionic functional group in the aqueous dispersion has a fiber width of 300 nm or less.

4. The hydrophobic cellulose nanofiber according to claim 2 or 3, wherein
the anionic functional group is selected from the group consisting of a carboxyl group, a carboxymethyl group, a sulfate ester group, a phosphate ester group, a phosphite ester group, and a xanthate ester group.

5. The hydrophobic cellulose nanofiber according to any one of claims 1 to 4, wherein
the isocyanate group-containing organopolysiloxane (B) is represented by the following general formula (1) or (2), 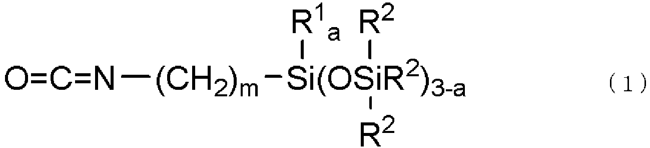 wherein R¹ is a monovalent alkyl group having 1 to 6 carbon atoms; R² is independently an alkyl group having 1 to 6 carbon atoms, a phenyl group, or a triorganosiloxy group represented by -OSiR³₃; R³ is independently a monovalent organic group having 1 to 6 carbon atoms; "m" is an integer of 1 to 10; and "a" is an integer of 0 to 1, 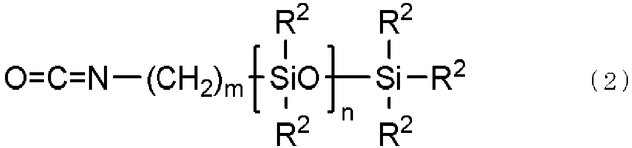 wherein R² is the same as the above; "m" is an integer of 1 to 10; and "n" is an integer of 1 to 500.

6. The hydrophobic cellulose nanofiber according to claim 5, wherein
the isocyanate group-containing organopolysiloxane represented by the general formula (1) is tris(trimethylsiloxy)silylpropyl isocyanate.

7. A hydrophobic cellulose nanofiber dispersion, in which the hydrophobic cellulose nanofiber according to any one of claims 1 to 6 is dispersed in an oil agent (C) .

8. The hydrophobic cellulose nanofiber dispersion according to claim 7, wherein the oil agent (C) is one or more kinds selected from the group consisting of aliphatic alcohols, silicone oils, and hydrocarbon oils.

9. The hydrophobic cellulose nanofiber dispersion according to claim 7 or 8, wherein
a mass ratio between the oil agent (C) and the hydrophobic cellulose nanofiber falls in 1:0.0001 to 1:0.5.

10. The hydrophobic cellulose nanofiber dispersion according to any one of claims 7 to 9, wherein
0.2 mass% oily dispersion of the hydrophobic cellulose nanofiber has a 600 nm light transmittance of 70% or more.

11. A cosmetic comprising the hydrophobic cellulose nanofiber dispersion according to any one of claims 7 to 10.

12. The cosmetic according to claim to 11, wherein
the hydrophobic cellulose nanofiber dispersion is used as an emulsified composition.
